# EUROPEAN PATENT APPLICATION

(11) **EP 3 819 370 A1**
(43) Date of publication of application: **12.05.2021**
(21) Application number: 19831123.5
(22) Date of filing: 28.06.2019
(51) Int. Cl.: C12M 3/02, C12M 1/00, C12M 1/02, C12M 1/34

(54) **CELL CULTURE DEVICE AND STIRRING METHOD**

(30) Priority: 05.07.2018 JP 2018128455
(71) Applicant: FUJIFILM Corporation, Tokyo 106-8620 (JP)
(72) Inventor: INADA, Atsushi, Ashigarakami-gun, Kanagawa 258-8577 (JP); KATOU, Nobuhiko, Ashigarakami-gun, Kanagawa 258-8577 (JP); TAKAYAMA, Hidetoshi, Ashigarakami-gun, Kanagawa 258-8577 (JP)
(74) Representative: Klunker IP Patentanwälte PartG mbB
(86) International application number: PCT/JP2019/025813
(87) International publication number: WO 2020/009017

(57) **Abstract**

A cell culture device includes plural three or more of culture vessels, a flow path that connects the plural culture vessels to each other, and a transfer control unit that performs transfer control to transfer a cell suspension housed in any of the plural culture vessels to any other of the plural culture vessels via the flow path.

## Description

### Technical Field

Priority is claimed on Japanese Patent Application No. 2018-128455, filed on July 5, 2018, the content of which is incorporated herein by reference.

The disclosed technique relates to a cell culture device and a stirring method.

### Background Art

For example, the following techniques are known as techniques related to a cell culture device.

For example, JP2002-531114A discloses a device that includes first and second culture vessels for housing a culture, a gas source, a culture medium source, and a germicide source, and further includes a conduit system which has means for connecting one of the two culture vessels to the culture medium source, selectively connecting the two culture vessels to each other, and selectively connecting the other culture vessel to the germicide source.

In addition, JP2015-188392A discloses a cell culture system that comprises a culture medium supply vessel which supplies a culture medium to a plurality of culture vessels and the culture vessels which have different culture areas as the plurality of culture vessels, in which the plurality of culture vessels each comprise one port for transferring a content to the other vessel, and the plurality of culture vessels are connected to the culture medium supply vessel using a tube, in ascending order of culture area.

### SUMMARY OF INVENTION

### Technical Problem

In a case where a large number of cells are housed and cultured in a culture vessel together with a culture medium, a cell suspension containing the cells and the culture medium is stirred using a stirring blade in order to distribute nutrients and oxygen which are necessary for cell proliferation throughout all of the cells. However, in a case where the cell suspension is stirred using the stirring blade, a shearing force acts on the cells, and accordingly the cells are damaged. As a result, there is a possibility that cell proliferative properties decrease or a survival rate decreases.

In large-scale culture, since nutrients and oxygen in the culture medium are likely to be biased, the importance of stirring processing is high, while energy necessary for stirring is great. Therefore, the cells are greatly damaged.

The disclosed technique is devised in view of such points, and an object thereof is to provide a cell culture device and a stirring method, which allow a cell suspension to be stirred while suppressing damage to cells.

### Solution to Problem

According to an aspect of the disclosed technique, there is provided a cell culture device including a plurality of three or more of culture vessels, a flow path that connects the plurality of culture vessels to each other, and a transfer control unit that performs transfer control to transfer a cell suspension housed in any of the plurality of culture vessels to any other of the plurality of culture vessels via the flow path.

Accordingly, it is possible to stir the cell suspension while suppressing damage to the cells.

The cell culture device according to the aspect of the disclosed technique may further include a housing vessel that houses the plurality of culture vessels.

As the housing vessel functions as an incubator, a preferable culture environment for the cells can be formed.

The cell culture device according to the aspect of the disclosed technique may further include a monitor device that is disposed at the flow path and monitors a cell contained in the cell suspension passing through the flow path.

Accordingly, it is possible to observe all of the cells contained in the cell suspension in parallel with stirring processing of the cell suspension.

The monitor device may include an imaging device that images the cell contained in the cell suspension passing through the flow path.

Accordingly, it is possible to acquire images of the cells contained in the cell suspension in parallel with stirring processing of the cell suspension.

It is preferable that the transfer control unit causes the cell suspension transferred by the transfer control to pass through a part of the flow path, at which the monitor device is disposed.

Accordingly, it is possible to observe all of the cells contained in the cell suspension in parallel with stirring processing of the cell suspension.

The transfer control unit determines, based on a state of the cell monitored by the monitor device, a period until a next transfer of the cell suspension containing the cell

Accordingly, in transfer control, it is possible to set an appropriate cycle time depending on the state of the cell.

There may be four or more culture vessels. The monitor device may include a flow cell that has a first flow port and a second flow port communicating with the first flow port. The flow path may include a first flow path connected to the first flow port and any two or more of the plurality of culture vessels and a second flow path connected to the second flow port and any other two or more of the plurality of culture vessels.

Accordingly, since one cycle of transfer control can be completed without reciprocating the cell suspension between the culture vessels, it is possible to efficiently perform stirring processing of the cell suspension.

In the transfer control, the transfer control unit may mix the cell suspensions housed in any two or more of the plurality of culture vessels by transferring the cell suspensions to another culture vessel of the plurality of culture vessels.

Accordingly, since mixing processing can be performed in parallel with stirring processing as for the cell suspension housed in the plurality of culture vessels, variations in cell quality between the culture vessels can be suppressed.

In the transfer control, the transfer control unit may perform transfer of the cell suspension through pressure control inside the culture vessels. In addition, the pressure control may be performed by introducing a gas into a culture vessel housing the cell suspension, which is a transfer target, out of the plurality of culture vessels.

Accordingly, it is possible to transfer the cell suspension while suppressing damage to the cells.

In the pressure control, as the gas introduced into the culture vessels, a gas whose temperature and composition are adjusted may be used.

Accordingly, a culture environment preferable for the cells can be formed.

The cell culture device according to the aspect of the disclosed technique may further include a heat transfer medium in contact with each of the plurality of culture vessels and a heat source that heats the heat transfer medium.

Accordingly, a temperature of the cell suspension housed in the culture vessel can be controlled to have a desired temperature, and a culture environment preferable for the cells can be formed.

According to another aspect of the disclosed technique, there is provided a stirring method of stirring a cell suspension housed in any of a plurality of three or more culture vessels of a cell culture device, which includes the plurality of culture vessels and a flow path that connects the plurality of culture vessels to each other. The stirring method includes stirring the cell suspension, which is a transfer target, by transferring the cell suspension housed in any of the plurality of culture vessels to any other of the plurality of culture vessels via the flow path.

Accordingly, it is possible to stir the cell suspension while suppressing damage to the cells.

The stirring method according to the aspect of the disclosed technique may further include monitoring a cell contained in the cell suspension, which is the transfer target, while the cell suspension passes through the flow path.

Accordingly, it is possible to observe all of the cells contained in the cell suspension in parallel with stirring processing of the cell suspension.

The stirring method according to the aspect of the disclosed technique may include determining, based on a state of the monitored cell, a period until a next transfer of the cell suspension containing the cell. In this case, as a size of a monitored cell aggregate is larger, shortening the period until the next transfer of the cell suspension containing the cell aggregate is preferable.

Accordingly, it is possible to set an appropriate cycle time depending on the state of the cell.

The stirring method according to the aspect of the disclosed technique may further include mixing the cell suspensions housed in any two or more of the plurality of culture vessels by transferring the cell suspensions to another culture vessel of the plurality of culture vessels.

Accordingly, since mixing processing can be performed in parallel with stirring processing as for the cell suspension housed in the plurality of culture vessels, variations in cell quality between the culture vessels can be suppressed.

### Effects of Invention

In the disclosed technique, it is possible to stir the cell suspension while suppressing damage to the cells.

### BRIEF DESCRIPTION OF THE DRAWINGS

Fig. 1 is a diagram showing an example of a configuration of a cell culture device according to an embodiment of the disclosed technique.
Fig. 2A is a diagram showing an example of transfer control according to the embodiment of the disclosed technique.
Fig. 2B is a diagram showing an example of the transfer control according to the embodiment of the disclosed technique.
Fig. 2C is a diagram showing an example of the transfer control according to the embodiment of the disclosed technique.
Fig. 2D is a diagram showing an example of the transfer control according to the embodiment of the disclosed technique.
Fig. 2E is a diagram showing an example of the transfer control according to the embodiment of the disclosed technique.
Fig. 2F is a diagram showing an example of the transfer control according to the embodiment of the disclosed technique.
Fig. 2G is a diagram showing an example of the transfer control according to the embodiment of the disclosed technique.
Fig. 3 is a timing chart showing an example of the transfer control according to the embodiment of the disclosed technique.
Fig. 4 is a flowchart showing an example of flow of processing carried out in a case where a transfer control unit performs the transfer control according to the embodiment of the disclosed technique.
Fig. 5 is a perspective view illustrating an example of a configuration of a heat transfer medium comprising a heater according to the embodiment of the disclosed technique.
Fig. 6 is a diagram showing an example of the configuration of the cell culture device according to the embodiment of the disclosed technique.
Fig. 7 is a diagram showing an example of the configuration of the cell culture device according to the embodiment of the disclosed technique.
Fig. 8A is a diagram showing an example of the transfer control according to the embodiment of the disclosed technique.
Fig. 8B is a diagram showing an example of the transfer control according to the embodiment of the disclosed technique.
Fig. 8C is a diagram showing an example of the transfer control according to the embodiment of the disclosed technique.
Fig. 8D is a diagram showing an example of the transfer control according to the embodiment of the disclosed technique.
Fig. 8E is a diagram showing an example of the transfer control according to the embodiment of the disclosed technique.
Fig. 9 is a timing chart showing an example of the transfer control according to the embodiment of the disclosed technique.
Fig. 10A is a diagram showing an example of the transfer control according to the embodiment of the disclosed technique.
Fig. 10B is a diagram showing an example of the transfer control according to the embodiment of the disclosed technique.
Fig. 10C is a diagram showing an example of the transfer control according to the embodiment of the disclosed technique.
Fig. 11A is a diagram showing an example of the transfer control according to the embodiment of the disclosed technique.
Fig. 11B is a diagram showing an example of the transfer control according to the embodiment of the disclosed technique.
Fig. 11C is a diagram showing an example of the transfer control according to the embodiment of the disclosed technique.
Fig. 12 is a diagram showing an example of the configuration of the cell culture device according to the embodiment of the disclosed technique.
Fig. 13A is a diagram showing an example of the transfer control according to the embodiment of the disclosed technique.
Fig. 13B is a diagram showing an example of the transfer control according to the embodiment of the disclosed technique.
Fig. 13C is a diagram showing an example of the transfer control according to the embodiment of the disclosed technique.
Fig. 13D is a diagram showing an example of the transfer control according to the embodiment of the disclosed technique.
Fig. 13E is a diagram showing an example of the transfer control according to the embodiment of the disclosed technique.
Fig. 13F is a diagram showing an example of the transfer control according to the embodiment of the disclosed technique.
Fig. 14 is a timing chart showing an example of the transfer control according to the embodiment of the disclosed technique.

### DESCRIPTION OF EMBODIMENTS

Hereinafter, embodiments of the present disclosure will be described with reference to the drawings. In each of the drawings, substantially the same or equivalent components or portions will be assigned with the same reference symbols.

### [First Embodiment]

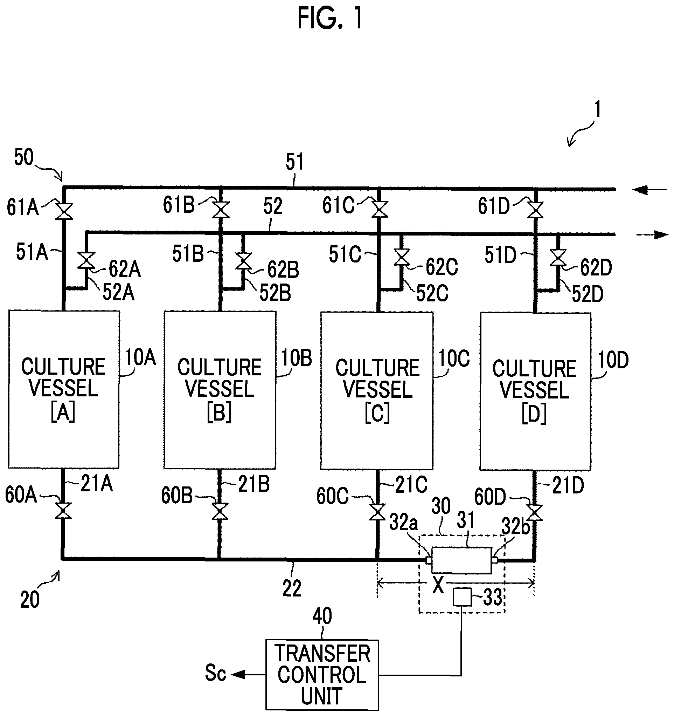

Fig. 1 is a diagram showing an example of a configuration of a cell culture device 1 according to a first embodiment of the disclosed technique. The cell culture device 1 is composed of a plurality of culture vessels 10A, 10B, 10C, and 10D, a monitor device 30, and a transfer control unit 40.

Each of the culture vessels 10A to 10D is a vessel that houses a cell suspension containing at least one of a plurality of cells or cell aggregates. The form of each of the culture vessels 10A to 10D is not particularly limited, and for example, it is possible to use a vessel made of glass or made of stainless steel or a vessel having a bag form composed of a plastic film having gas permeability. Although a configuration including the four culture vessels 10A to 10D is exemplified in the example shown in Fig. 1, the cell culture device 1 may include three or five or more culture vessels.

The culture vessels 10A to 10D are connected to each other by a flow path 20. The flow path 20 is composed of individual flow paths 21A, 21B, 21C, and 21D, which are provided to correspond to the culture vessels 10A to 10D respectively, and a common flow path 22 that connects the individual flow paths 21A to 21D to each other. Valves 60A, 60B, 60C, and 60D are provided in the middle of the individual flow paths 21A to 21D, respectively.

The cell culture device 1 has a pressure adjusting mechanism 50 for adjusting pressures inside the culture vessels 10A to 10D. The pressure adjusting mechanism 50 has a gas introduction pipe 51 through which a gas introduced into the culture vessels 10A to 10D flows and a gas discharge pipe 52 through which a gas discharged from the inside of the culture vessels 10A to 10D flows. The gas introduction pipe 51 has individual pipes 51A, 51B, 51C and 51D, which are provided to correspond to the culture vessels 10A to 10D respectively. Valves 61A, 61B, 61C, and 61D are provided in the middle of the individual pipes 51A to 51D, respectively. The gas discharge pipe 52 has individual pipes 52A, 52B, 52C and 52D, which are provided to correspond to the culture vessels 10A to 10D respectively. Valves 62A, 62B, 62C, and 62D are provided in the middle of the individual pipes 52A to 52D, respectively. As the pressure adjusting mechanism 50 adjusts the balance of pressures inside the culture vessels 10A to 10D, the cell suspension housed therein is transferred between the culture vessels 10A to 10B. As a gas introduced into the culture vessels 10A to 10D, a gas whose temperature and composition are adjusted may be used. Accordingly, it is possible to form a preferable culture environment in the culture vessels 10A to 10D.

The monitor device 30 is provided in a section X between a connection portion of the common flow path 22 to the individual flow path 21C and a connection portion of the common flow path to the individual flow path 21D. The monitor device 30 monitors the cells contained in the cell suspension passing through the section X of the common flow path 22. The monitor device 30 is composed of a flow cell 31 and an imaging device 33.

The flow cell 31 is entirely composed of a material having light transmittance such as glass and plastic. The flow cell 31 has a first flow port 32a and a second flow port 32b communicating with the first flow port 32a.

The imaging device 33 has an imaging field of view set in a region between the first flow port 32a and the second flow port 32b of the flow cell 31, and continuously images cells (cell aggregates), which are contained in the cell suspension flowing from one of the first flow port 32a or the second flow port 32b toward the other, through the flow cell 31. A plurality of images captured by the imaging device 33 are transmitted to the transfer control unit 40.

The transfer control unit 40 performs transfer control of transferring the cell suspension housed in any of the culture vessels 10A to 10D to any other of the culture vessels via the flow path 20. The transfer control unit 40 supplies a control signal Sc to the valves 60A to 60D, the valves 61A to 61D, and the valves 62A to 62D, and selectively opens and closes the valves to perform the transfer control. Through a liquid flow of the cell suspension transferred through the transfer control, the cell suspension is stirred, and thus nutrients and oxygen contained in a culture medium can be distributed throughout all of the cells. The transfer control unit 40 performs the transfer control such that the cell suspension passes through the section X of the common flow path 22, in which the monitor device 30 is disposed.

The transfer control unit 40 intermittently performs transfer of the cell suspension for each of the culture vessels 10A to 10D. The transfer control unit 40 determines a period until the next transfer of the cell suspension containing the cells (cell aggregates) based on the state of the cells (cell aggregates) monitored by the monitor device 30. More specifically, the transfer control unit 40 derives an average size of a plurality of cell aggregates contained in the cell suspension, which is a transfer target, from the plurality of images supplied from the imaging device 33 that composing the monitor device 30. The transfer control unit 40 determines a period until the next transfer of the cell suspension containing the cell aggregates based on the derived average size of the cell aggregates.

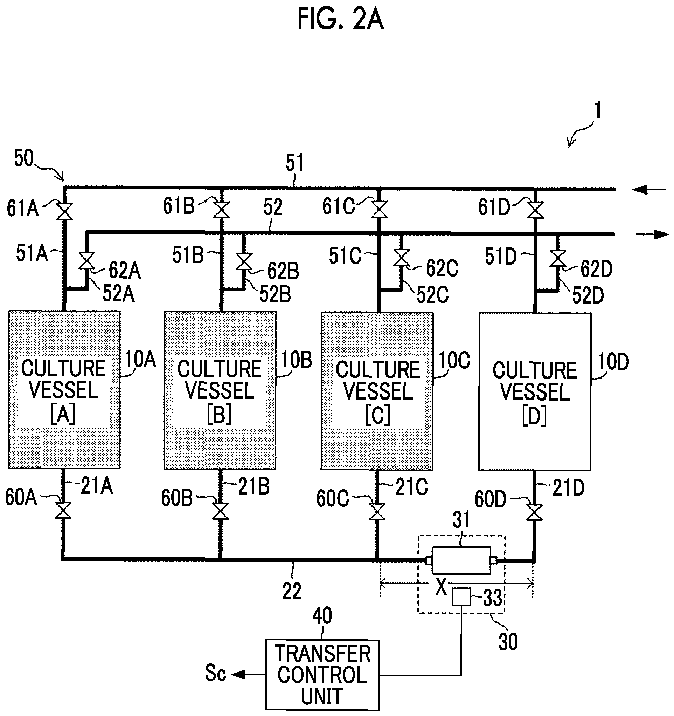

Hereinafter, an example of transfer control by the transfer control unit 40 will be described with reference to Figs. 2A to 2G.

In an initial state, as shown in Fig. 2A, the culture vessels 10A, 10B, and 10C each house a cell suspension containing a plurality of cells (cell aggregates), and the culture vessel 10D is in an empty state. The plurality of cells (cell aggregates) are suspended in a stationary state in culture media in the culture vessels 10A, 10B, and 10C. A thickener may be added to each culture medium in order to cause the plurality of cells (cell aggregates) to be suspended in the culture medium. In addition, in the initial state, each of the valves 60A to 60D, 61A to 61D, and 62A to 62D is in a closed state.

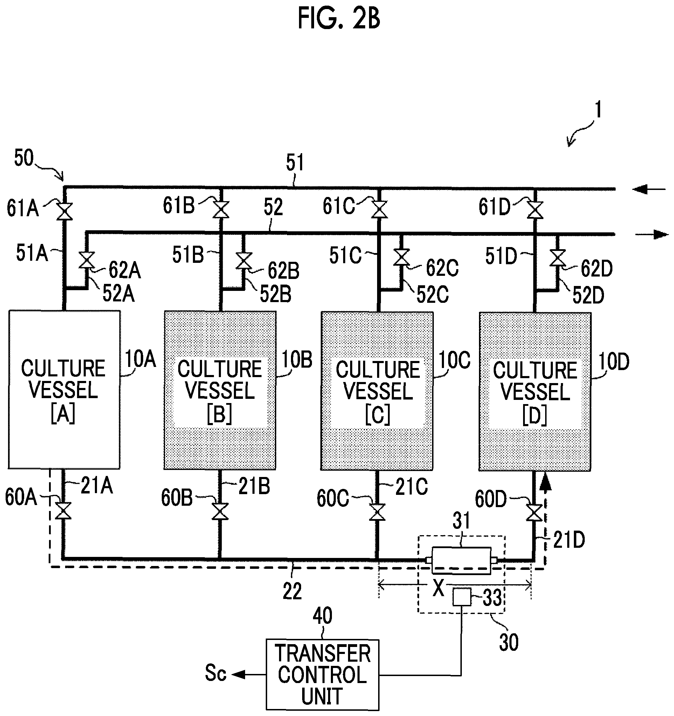

By supplying the control signal Sc to each of the valves 60A, 60D, 61A, and 62D, the transfer control unit 40 controls the valves such that the valves are in an open state. Accordingly, a gas is introduced into the culture vessel 10A via the gas introduction pipe 51, and the inside of the culture vessel 10A is pressurized. As shown in Fig. 2B, the cell suspension housed inside the culture vessel 10A is transferred to the culture vessel 10D via the individual flow path 21A and the common flow path 22. While the cell suspension passes through the section X of the common flow path 22, the cells contained in the cell suspension are monitored by the monitor device 30. That is, the imaging device 33 that composes the monitor device 30 continuously images the cells contained in the cell suspension passing through the flow cell 31. The imaging device 33 performs imaging, for example, at an interval that allows imaging all of the cells (cell aggregates) contained in the cell suspension, which is a transfer target. The imaging device 33 may image some cells (cell aggregates) contained in the cell suspension, which is a transfer target. Images of the cells captured by the imaging device 33 are supplied to the transfer control unit 40. The transfer control unit 40 controls the valves 60A, 60D, 61A, and 62D such that the valves are in a closed state after the transfer of the cell suspension is completed.

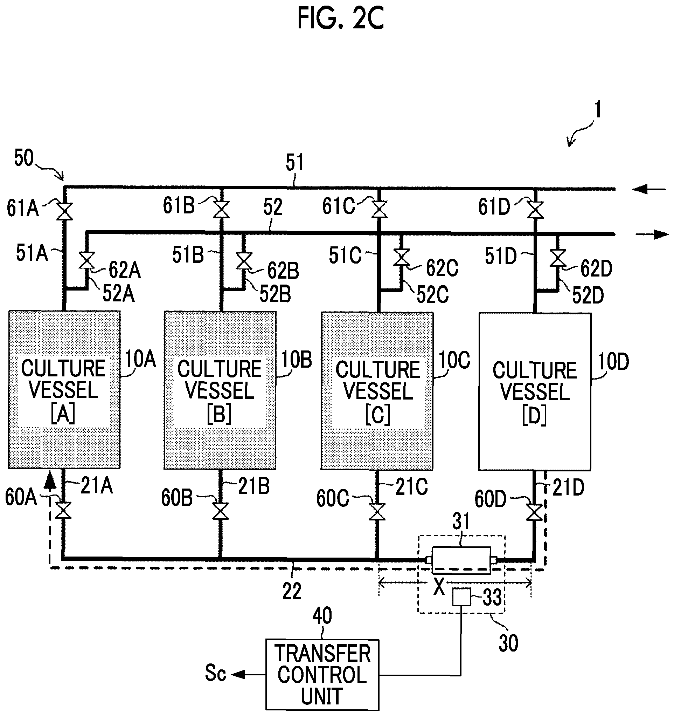

Next, the transfer control unit 40 controls the valves 60A, 60D, 62A, and 61D such that the valves are in an open state. Accordingly, a gas is introduced into the culture vessel 10D via the gas introduction pipe 51, and the inside of the culture vessel 10D is pressurized. As shown in Fig. 2C, the cell suspension housed inside the culture vessel 10D is transferred to the culture vessel 10A via the individual flow path 21D and the common flow path 22. While the cell suspension passes through the section X of the common flow path 22. the cells contained in the cell suspension are monitored by the monitor device 30. The transfer control unit 40 controls the valves 60A, 60D, 62A, and 61D such that the valves are in a closed state after the transfer of the cell suspension is completed.

As described above, in the initial state, the transfer control unit 40 performs transfer control of transferring the cell suspension housed in the culture vessel 10A to the culture vessel 10D and then returning the cell suspension to the culture vessel 10A. Accordingly, the transferred cell suspension is stirred, and the nutrients and the oxygen which are contained in the culture medium are distributed throughout all of the cells. In a case where the cell suspension returns from the culture vessel 10D to the culture vessel 10A, the monitoring of the cells by the monitor device 30 may be omitted.

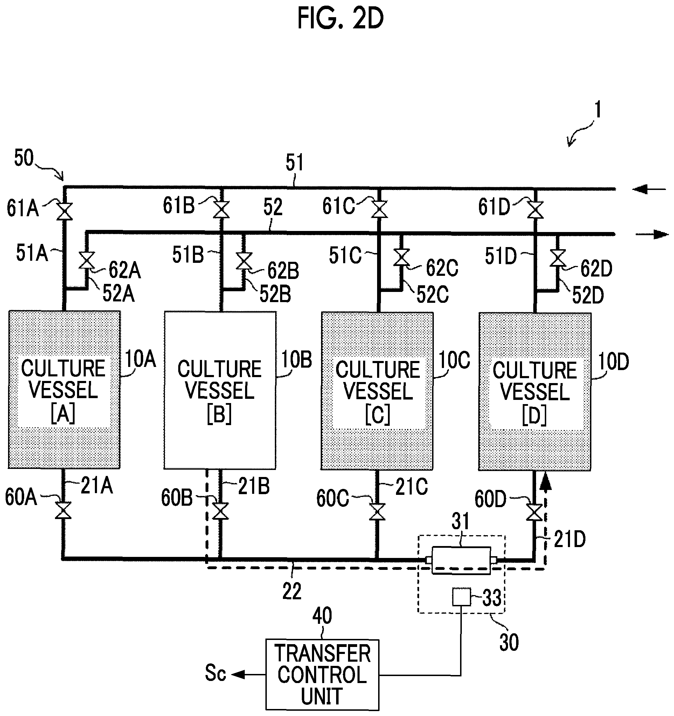

Next, by supplying the control signal Sc to each of the valves 60B, 60D, 61B, and 62D, the transfer control unit 40 controls the valves such that the valves are in an open state. Accordingly, a gas is introduced into the culture vessel 10B via the gas introduction pipe 51, and the inside of a culture vessel B is pressurized. As shown in Fig. 2D, the cell suspension housed inside the culture vessel 10B is transferred to the culture vessel 10D via the individual flow path 21B and the common flow path 22. While the cell suspension passes through the section X of the common flow path 22, the cells contained in the cell suspension are monitored by the monitor device 30. The transfer control unit 40 controls the valves 60B, 60D, 61B, and 62D such that the valves are in a closed state after the transfer of the cell suspension is completed.

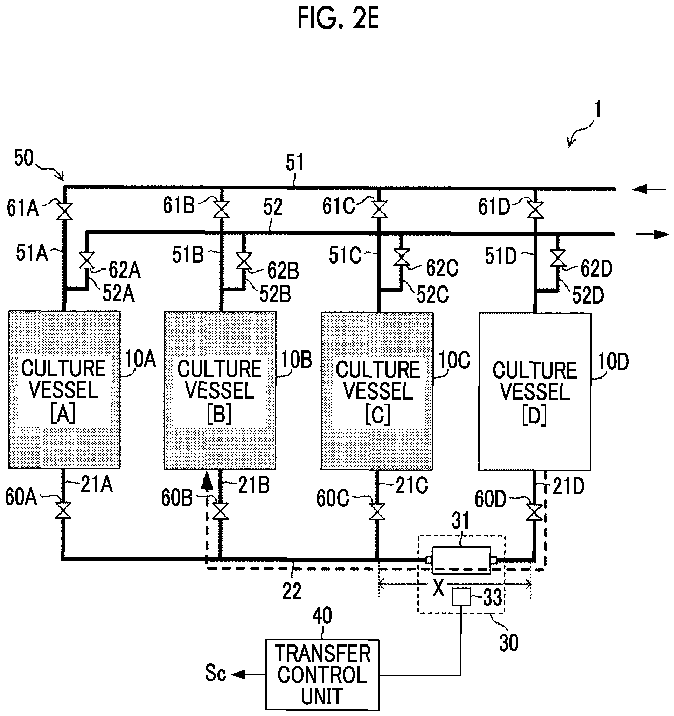

Next, the transfer control unit 40 controls the valves 60B, 60D, 62B, and 61D such that the valves are in an open state. Accordingly, a gas is introduced into the culture vessel 10D via the gas introduction pipe 51, and the inside of the culture vessel 10D is pressurized. As shown in Fig. 2E, the cell suspension housed inside the culture vessel 10D is transferred to the culture vessel 10B via the individual flow path 21D and the common flow path 22. While the cell suspension passes through the section X of the common flow path 22, the cells contained in the cell suspension are monitored by the monitor device 30. The transfer control unit 40 controls the valves 60B, 60D, 62B, and 61D such that the valves are in a closed state after the transfer of the cell suspension is completed.

As described above, in the initial state, the transfer control unit 40 performs transfer control of transferring the cell suspension housed in the culture vessel 10B to the culture vessel 10D and then returning the cell suspension to the culture vessel 10B. Accordingly, the transferred cell suspension is stirred, and the nutrients and the oxygen which are contained in the culture medium are distributed throughout all of the cells. In a case where the cell suspension returns from the culture vessel 10D to the culture vessel 10B, the monitoring of the cells by the monitor device 30 may be omitted.

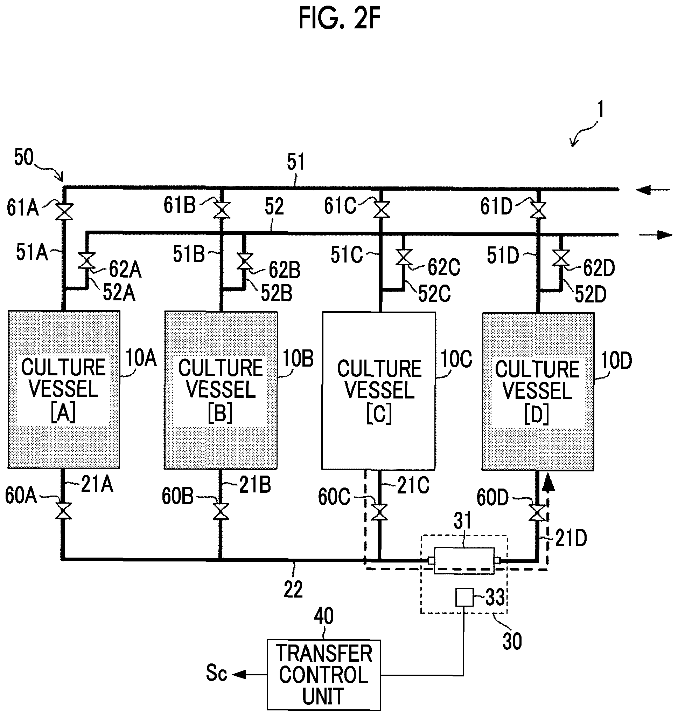

Next, by supplying the control signal Sc to each of the valves 60C, 60D, 61C, and 62D, the transfer control unit 40 controls the valves such that the valves are in an open state. Accordingly, a gas is introduced into the culture vessel 10C via the gas introduction pipe 51, and the inside of a culture vessel C is pressurized. As shown in Fig. 2F, the cell suspension housed inside the culture vessel 10C is transferred to the culture vessel 10D via the individual flow path 21C and the common flow path 22. While the cell suspension passes through the section X of the common flow path 22, the cells contained in the cell suspension are monitored by the monitor device 30. The transfer control unit 40 controls the valves 60C, 60D, 61C, and 62D such that the valves are in a closed state after the transfer of the cell suspension is completed.

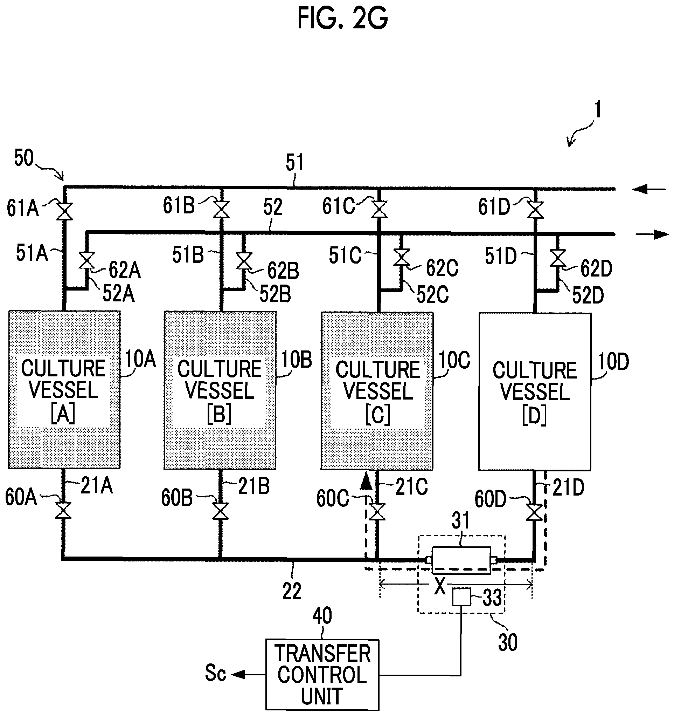

Next, the transfer control unit 40 controls the valves 60C, 60D, 62C, and 61D such that the valves are in an open state. Accordingly, a gas is introduced into the culture vessel 10D via the gas introduction pipe 51, and the inside of the culture vessel 10D is pressurized. As shown in Fig. 2G, the cell suspension housed inside the culture vessel 10D is transferred to the culture vessel 10C via the individual flow path 21D and the common flow path 22. While the cell suspension passes through the section X of the common flow path 22, the cells contained in the cell suspension are monitored by the monitor device 30. The transfer control unit 40 controls the valves 60C, 60D, 62C, and 61D such that the valves are in a closed state after the transfer of the cell suspension is completed.

As described above, in the initial state, the transfer control unit 40 performs transfer control of transferring the cell suspension housed in the culture vessel 10C to the culture vessel 10D and then returning the cell suspension to the culture vessel IOC. Accordingly, the transferred cell suspension is stirred, and the nutrients and the oxygen which are contained in the culture medium are distributed throughout all of the cells. In a case where the cell suspension returns from the culture vessel 10D to the culture vessel 10C, the monitoring of the cells by the monitor device 30 may be omitted.

As described above, in the cell culture device 1 according to the embodiment, the transfer of the cell suspension is performed for each culture vessel, and the transfer of the cell suspension housed in one culture vessel is intermittently performed.

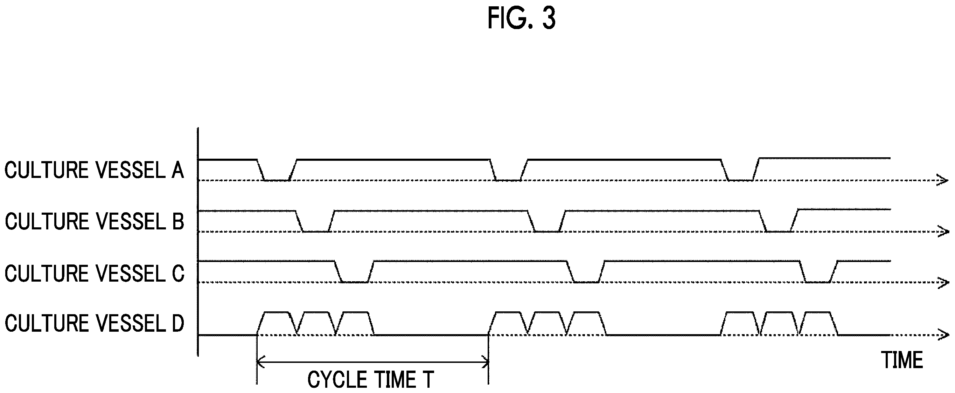

Fig. 3 is a timing chart corresponding to the series of processes of transfer control shown in Figs. 2A to 2G. In Fig. 3, "presence" and "absence" of the cell suspension are shown for each culture vessel. In Fig. 3, the "presence" of the cell suspension corresponds to a high level and the "absence" of the cell suspension corresponds to a low level. As shown in Fig. 3, the transfer control unit 40 repeatedly carries out transfer control in which one round-trip transfer of the cell suspension between each of the culture vessels 10A, 10B, and 10C and the culture vessel 10D is set as one set. The transfer control unit 40 determines, based on the state of cells (cell aggregates) monitored by the monitor device 30, a period (cycle time T) until the next transfer of the cell suspension housed in the culture vessels 10A, 10B, and 10C.

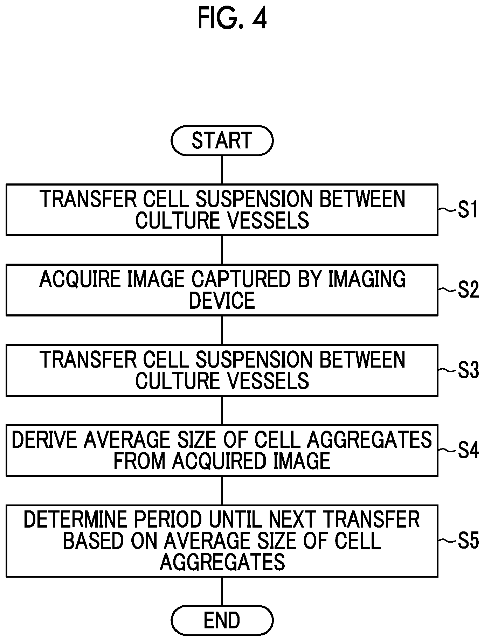

Fig. 4 is a flowchart showing an example of flow of processing carried out by the transfer control unit, for example, in a case of performing transfer control on the cell suspension housed in the culture vessel 10A.

In Step S1, by supplying the control signal Sc to each of the valves 60A, 60D, 61A, and 62D, the transfer control unit 40 controls the valves such that the valves are in an open state. Accordingly, the cell suspension housed inside the culture vessel 10A is transferred to the culture vessel 10D. While the cell suspension passes through the section X of the common flow path 22, the cells (cell aggregates) contained in the cell suspension are imaged by the imaging device 33. The transfer control unit 40 controls the valves 60A, 60D. 61A, and 62D such that the valves are in a closed state after the transfer of the cell suspension is completed.

In Step S2, the transfer control unit 40 acquires a plurality of images of the cells captured by the imaging device 33.

In Step S3, the transfer control unit 40 controls the valves 60A, 60D, 62A, and 63D such that the valves are in an open state. Accordingly, the cell suspension housed inside the culture vessel 10D is transferred to the culture vessel 10A. The transfer control unit 40 controls the valves 60A, 60D, 62A, and 61D such that the valves are in a closed state after the transfer of the cell suspension is completed.

In Step S4, the transfer control unit 40 derives an average size of the cell aggregates contained in the cell suspension from the plurality of images acquired in Step S2. For example, as for each of the cell aggregates contained in the cell suspension, an arithmetic mean value of diameters obtained by approximating a spherical shape may be applied as the average size of the cell aggregates.

In Step S5, the transfer control unit 40 derives the cycle time T corresponding to the average size of the cell aggregates derived in Step S4, for example, by referring to the table exemplified as Table 1 below. In the table exemplified as Table 1, a cycle time T1 (for example, 6 hours to 12 hours) is assigned to an average size of cell aggregates of 100 µm or more and less than 150 µm, a cycle time T2 (for example, 4 hours to 10 hours) is assigned to an average size of cell aggregates of 150 µm or more and less than 250 µm, and a cycle time T3 (2 hours to 8 hours) is assigned to an average size of cell aggregates of 250 µm or more and less than 500 µm. A relationship of T1 ≥ T2 ≥ T3 is satisfied. The transfer control unit 40 determines the cycle time T derived by referring to the table as a period until the next transfer of the cell suspension housed in the culture vessel 10A.

**[Table 1]**

| Average diameter of cell aggregates | Cycle time |
|---|---|
| 100 to 150 µm | T1 |
| 150 to 250 µm | T2 |
| 250 to 500 µm | T3 |

The transfer control unit 40 also carries out the same processing as described above also even a case of performing transfer control on the cell suspensions housed in the culture vessels 10B and 10C. In a case where it is assumed that the average size of the cell aggregates contained in the cell suspension housed in each of the culture vessels 10A to 10C is approximately the same, the cycle time T derived based on the average size of the cell aggregates contained in the cell suspension housed in any of the culture vessels 10A to 10C may be applied in transfer control of the cell suspension housed in another culture vessel.

As described above, in the cell culture device 1 according to the embodiment of the disclosed technique, the cell suspension is stirred as the cell suspension is transferred between the culture vessels 10A, 10B, and 10C and the culture vessel 10D. Accordingly, the nutrients and the oxygen which are contained in the culture medium are distributed throughout all of the cells. By stirring the cell suspension through the transfer of the cell suspension, damage to the cells can be reduced compared to a case where stirring is performed using a stirring blade. That is, in the cell culture device 1 according to the embodiment of the disclosed technique, it is possible to stir the cell suspension while suppressing damage to the cells.

In addition, in the cell culture device 1 according to the embodiment of the disclosed technique, the monitor device 30 is disposed in the section X of the common flow path 22, and the cells (cell aggregates) contained in the cell suspension, which is a transfer target, is monitored by the monitor device 30 while the cell suspension is being transferred. Accordingly, it is possible to observe all of the cells (cell aggregates) contained in the cell suspension in parallel with stirring processing of the cell suspension.

In addition, the cycle time T of transfer control is determined depending on the state of the cells (cell aggregates) monitored by the monitor device 30. More specifically, in a case where an average size of the cell aggregates derived from the images captured by the imaging device 33 is relatively small, a relatively long period is set as the cycle time T of transfer control. On the other hand, in a case where an average size of the cell aggregates derived from the images captured by the imaging device 33 is relatively large, a relatively long period is set as the cycle time T of transfer control. The cell aggregates, which are relatively small in size, have relatively low resistance to a shear force caused by the liquid flow of the cell suspension, and require a relatively small amount of nutrient and oxygen. On the other hand, the cell aggregates, which are relatively large in size, have relatively high resistance to a shear force caused by the liquid flow of the cell suspension, and require a relatively large amount of nutrient and oxygen. Therefore, as described above, the cycle time T of transfer control (stirring processing) suitable for the sizes of the cell aggregates can be set by determining the cycle time T of transfer control depending on the average size of the cell aggregates.

The cell monitoring results obtained by the monitor device 30 may be used for purposes other than the setting of the cycle time T of transfer control. For example, a timing at which culture medium replacement or passage processing is carried out may be determined based on images of the cells (cell aggregates) captured by the imaging device 33. In addition, the qualities of the cells may be determined based on the images of the cells (cell aggregates) captured by the imaging device 33.

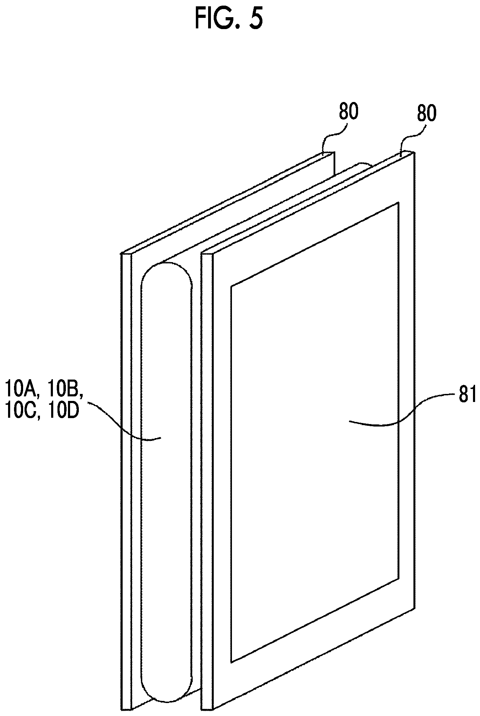

In addition, as illustrated in Fig. 5, the cell culture device 1 may comprise a heat transfer medium 80 in contact with each of the culture vessels 10A to 10D and a heater (heat source) 81 that heats the heat transfer medium 80. The heat transfer medium 80 is composed of a pair of metal or resin plate-shaped members each having a relatively high thermal conductivity, and each of the culture vessels 10A to 10D is sandwiched between the pair of plate-shaped members. The pair of plate-shaped members are configured such that an interval therebetween is constant. The heater 81 is attached to a surface of the heat transfer medium 80, which is opposite to a contact surface of the culture vessels 10A to 10D. Heat generated from a heater 80 is transmitted to the cell suspensions housed inside the culture vessels 10A to 10D via the heat transfer medium 80. A temperature of the heater 81 is controlled such that the cell suspensions housed in the culture vessels 10A to 10D maintain a desired temperature (for example, 37°C).

As described above, as the culture vessels 10A to 10D are sandwiched between the pair of plate-shaped members that compose the heat transfer medium 80, temperatures of the cell suspensions housed in the culture vessels 10A to 10D can be controlled to be a desired temperature, and a culture environment preferable for the cells can be formed. In addition, in a case where the culture vessels 10A to 10D each have a bag form composed of, for example, a plastic film, when transferring the cell suspension, the volume of the culture vessel expands as the inside of the culture vessel housing the cell suspension, which is a transfer target, is pressurized. As a result, it is difficult to perform pressure control inside the culture vessel, and it is difficult to perform dispensing control of the cell suspension. As the culture vessels 10A to 10D are sandwiched between the pair of plate-shaped members composing the heat transfer medium 80, the expansion of the volume in a case where the inside of each of the culture vessels 10A to 10D is pressurized can be restrained. Therefore, it is easy to perform pressure control inside the culture vessel, and it is easy to perform dispensing control of the cell suspension.

### [Second Embodiment]

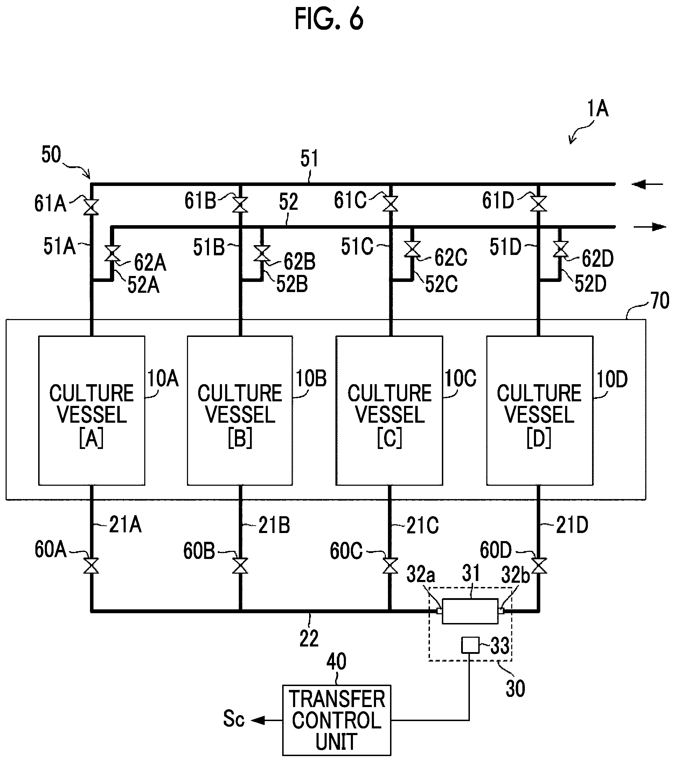

Fig. 6 is a diagram showing an example of a configuration of a cell culture device 1A according to a second embodiment of the disclosed technique.

The cell culture device 1A includes a housing vessel 70 that houses the culture vessels 10A to 10D. The housing vessel 70 has a function as an incubator, and has a temperature control mechanism that maintains an internal temperature of the housing vessel 70 constant (for example, 37°C). In addition, in a case where the culture vessels 10A to 10D each are in a bag form composed of a plastic film having gas permeability, a gas which contains oxygen and carbon dioxide and of which composition is adjusted is introduced into the housing vessel 70. Accordingly, oxygen and carbon dioxide which are necessary for cell proliferation can be incorporated into the cell suspensions housed in the culture vessels 10A to 10D. In the embodiment, the individual flow paths 21A to 21D, the common flow path 22, the gas introduction pipe 51, the gas discharge pipe 52, the monitor device 30, and the transfer control unit 40 are provided outside the housing vessel 70.

In the cell culture device 1A according to the embodiment, since the culture vessels 10A to 10D are housed inside the housing vessel 70 functioning as an incubator, a preferable culture environment for the cells housed in the culture vessels 10A to 10D can be formed.

### [Third Embodiment]

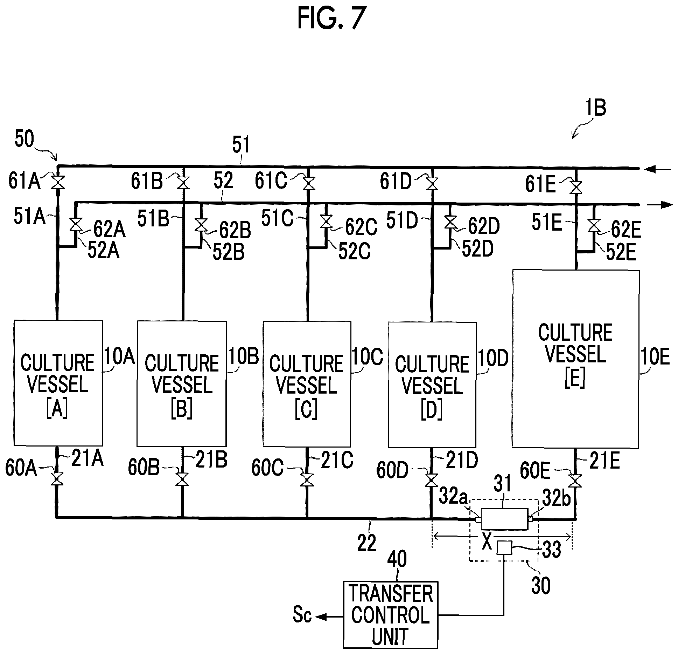

Fig. 7 is a diagram showing an example of a configuration of a cell culture device 1B according to a third embodiment of the disclosed technique.

The cell culture device 1B comprises a culture vessel 10E having a volume larger than the volume of each of the culture vessels 10A to 10D. The culture vessel 10E is connected to the common flow path 22 via an individual flow path 21E. A valve 60E is provided in the middle of the individual flow path 21E. The gas introduction pipe 51 has an individual pipe 51E provided to correspond to the culture vessel 10E. A valve 61E is provided in the middle of the individual pipe 51E. The gas discharge pipe 52 has an individual pipe 52E provided to correspond to the culture vessel 10E. A valve 62E is provided in the middle of the individual pipe 52E. The monitor device 30 is provided in the section X between a connection portion of the common flow path 22 to the individual flow path 21D and a connection portion of the common flow path to the individual flow path 21E.

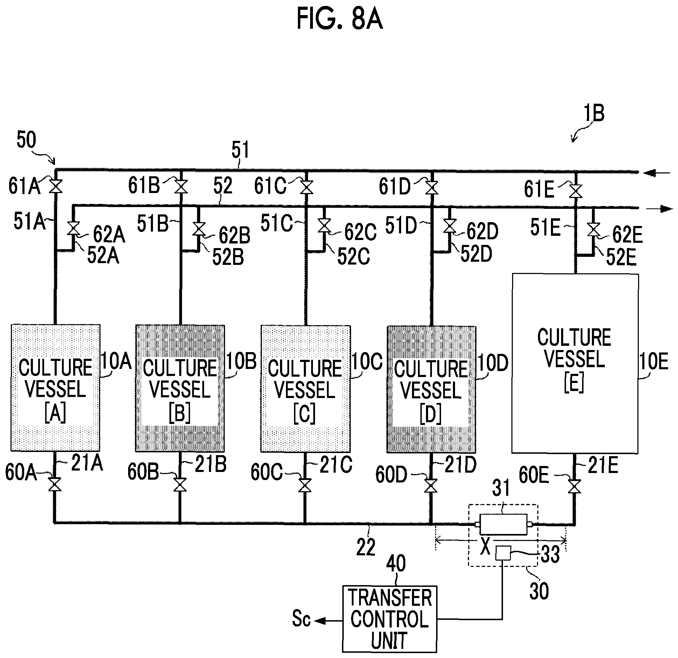

Figs. 8A to 8E are diagrams showing examples of transfer control by the transfer control unit 40 of the cell culture device 1B according to the embodiment.

In an initial state, as shown in Fig. 8A, the culture vessels 10A to 10D each house a cell suspension containing a plurality of cells (cell aggregates), and the culture vessel 10E is in an empty state. The plurality of cells (cell aggregates) are suspended in a stationary state in culture media in the culture vessels 10A to 10D. In addition, in the initial state, each of the valves 60A to 60E, 61

A to 61E, and 62A to 62E are in a closed state.

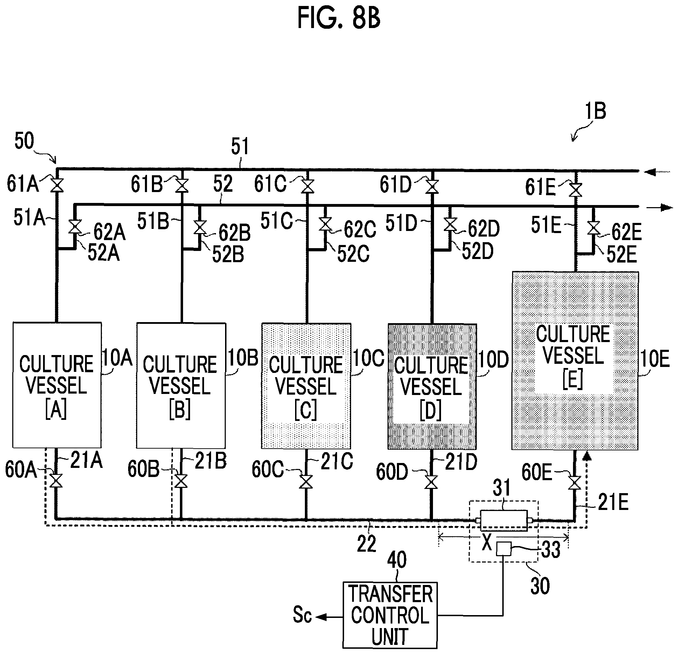

By supplying the control signal Sc to each of the valves 60A, 60B, 60E, 61A, 61B, and 62E, the transfer control unit 40 controls the valves such that the valves are in an open state. Accordingly, a gas is introduced into the culture vessels 10A and 10B via the gas introduction pipe 51, and the insides of the culture vessels 10A and 10B are pressurized. As shown in Fig. 8B, the cell suspensions housed inside the culture vessels 10A and 10B are transferred to the culture vessel 10E. While the cell suspensions pass through the section X of the common flow path 22, the cells contained in the cell suspensions are monitored by the monitor device 30. That is, the imaging device 33 that composes the monitor device 30 continuously images the cells contained in the cell suspensions passing through the flow cell 31. The imaging device 33 performs imaging, for example, at an interval that allows imaging all of the cells (cell aggregates) contained in the cell suspensions, which are transfer targets. Images of the cells captured by the imaging device 33 are supplied to the transfer control unit 40. The transfer control unit 40 controls the valves 60A, 60B, 60E, 61A, 61B, and 62E such that the valves are in a closed state after the transfers of the cell suspensions are completed.

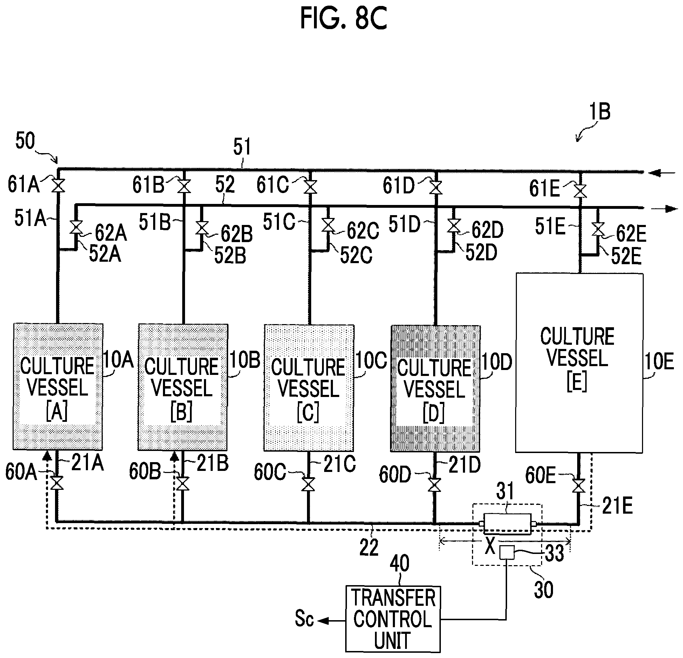

Next, the transfer control unit 40 controls the valves 60A, 60B, 60E, 62A, 62B, and 61E such that the valves are in an open state. Accordingly, a gas is introduced into the culture vessel 10E via the gas introduction pipe 51, and the inside of the culture vessel 10E is pressurized. As shown in Fig. 8C, the cell suspensions housed inside the culture vessel 10E are transferred to the culture vessels 10A and 10B. While the cell suspension passes through the section X of the common flow path 22, the cells contained in the cell suspension are monitored by the monitor device 30. The transfer control unit 40 controls the valves 60A, 60B, 60E, 62A, 62B, and 61E such that the valves are in a closed state after the transfers of the cell suspensions are completed.

As described above, in the initial state, the transfer control unit 40 performs transfer control of transferring the cell suspensions housed in the culture vessels 10A and 10B to the culture vessel 10E and then returning the cell suspensions to the culture vessels 10A and 10B. Accordingly, the transferred cell suspensions are stirred, and the nutrients and the oxygen which are contained in the culture media are distributed throughout all of the cells. In addition, in the initial state, the cell suspensions housed in the culture vessels 10A and 10B are mixed in the culture vessel 10E, which is a transfer destination. Although a case where the transfer of the cell suspension between the culture vessel 10A and the culture vessel 10E and the transfer of the cell suspension between the culture vessel 10B and the culture vessel 10E are performed simultaneously is exemplified in the embodiment, the transfers may be performed in turn. In addition, in a case where the cell suspensions return from the culture vessel 10E to the culture vessels 10A and 10B, the monitoring of the cells by the monitor device 30 may be omitted.

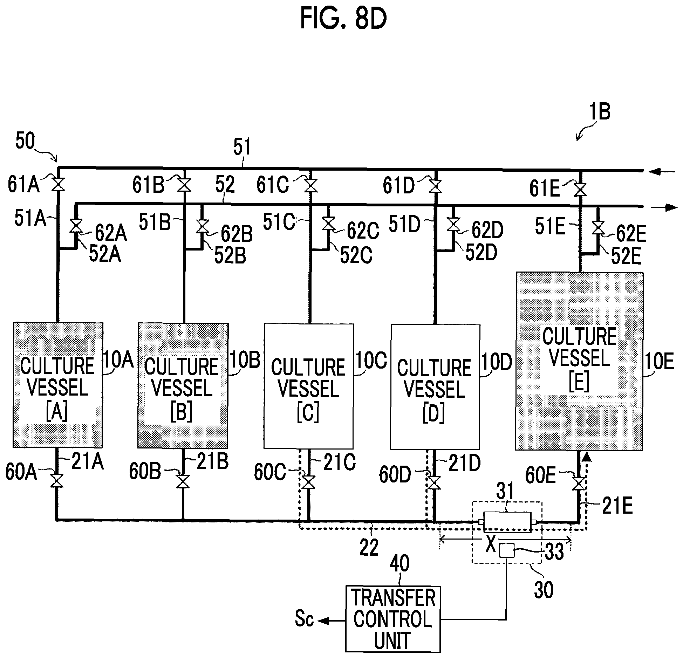

Next, by supplying the control signal Sc to each of the valves 60C, 60D, 60E, 61C, 61D, and 62E, the transfer control unit 40 controls the valves such that the valves are in an open state. Accordingly, a gas is introduced into the culture vessels 10C and 10D via the gas introduction pipe 51, and the insides of the culture vessels 10C and 10D are pressurized. As shown in Fig. 8D, the cell suspensions housed inside the culture vessels 10C and 10D are transferred to the culture vessel 10E. While the cell suspensions pass through the section X of the common flow path 22, the cells contained in the cell suspensions are monitored by the monitor device 30. The transfer control unit 40 controls the valves 60C, 60D, 60E, 61C, 61D, and 62E such that the valves are in a closed state after the transfers of the cell suspensions are completed.

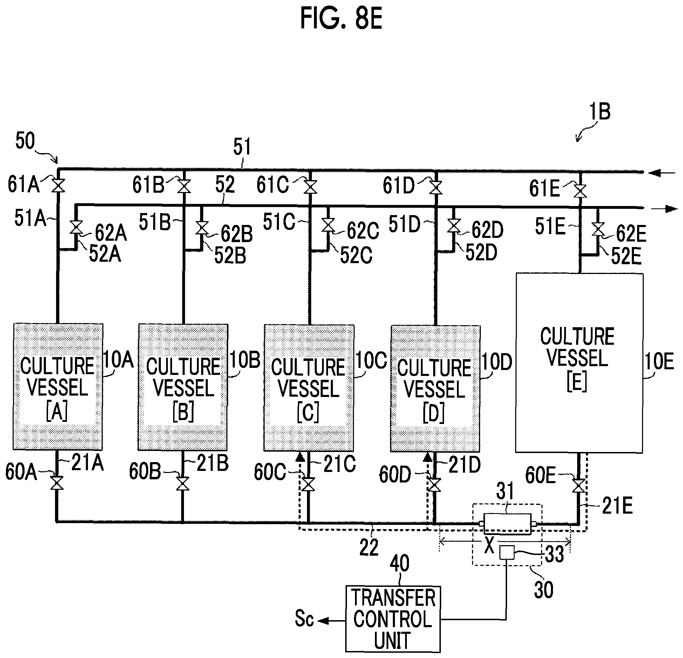

Next, the transfer control unit 40 controls the valves 60C, 60D, 60E, 62C, 62D, and 61E such that the valves are in an open state. Accordingly, a gas is introduced into the culture vessel 10E via the gas introduction pipe 51, and the inside of the culture vessel 10D is pressurized. As shown in Fig. 8E, the cell suspensions housed inside the culture vessel 10E are transferred to the culture vessels 10C and 10D. While the cell suspension passes through the section X of the common flow path 22, the cells contained in the cell suspension are monitored by the monitor device 30. The transfer control unit 40 controls the valves 60C, 60D, 60E, 62C, 62D, and 61E such that the valves are in a closed state after the transfers of the cell suspension are completed.

As described above, in the initial state, the transfer control unit 40 performs transfer control of transferring the cell suspensions housed in the culture vessels 10C and 10D to the culture vessel 10E and then returning the cell suspensions to the culture vessels 10C and 10D. Accordingly, the transferred cell suspensions are stirred, and the nutrients and the oxygen which are contained in the culture media are distributed throughout all of the cells. In addition, in the initial state, the cell suspensions housed in the culture vessels 10C and 10D are mixed in the culture vessel 10E, which is a transfer destination. Although a case where the transfer of the cell suspension between the culture vessel 10C and the culture vessel 10E and the transfer of the cell suspension between the culture vessel 10D and the culture vessel 10E are performed simultaneously is exemplified in the embodiment, the transfers may be performed in turn. In addition, in a case where the cell suspensions return from the culture vessel 10E to the culture vessels 10C and 10D, the monitoring of the cells by the monitor device 30 may be omitted.

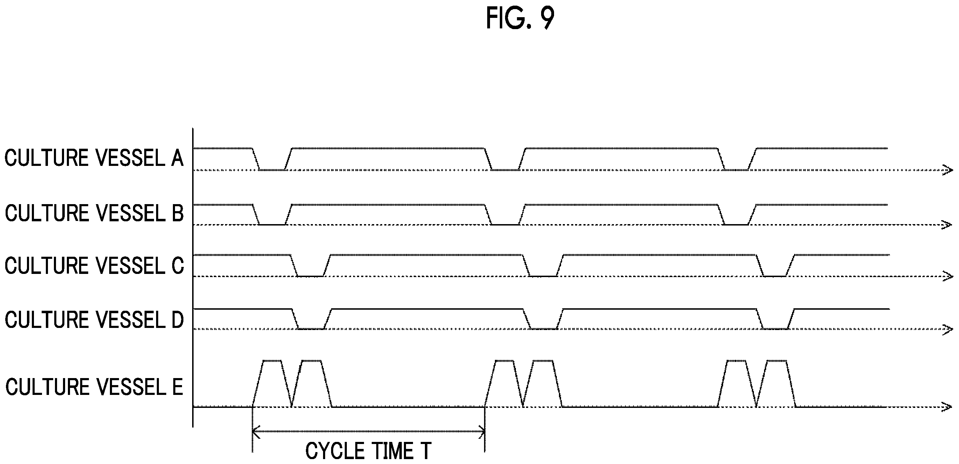

Fig. 9 is a timing chart corresponding to the series of processes of transfer control shown in Figs. 8A to 8E. In Fig. 9, "presence" and "absence" of the cell suspension are shown for each culture vessel. In Fig. 9, the "presence" of the cell suspension corresponds to a high level and the "absence" of the cell suspension corresponds to a low level.

As shown in Fig. 9, the transfer control unit 40 repeatedly carries out transfer control in which one round-trip transfer of the cell suspension between each of the culture vessels 10A to 10D and the culture vessel 10E is set as one set. The transfer control unit 40 determines, based on the state of cells monitored by the monitor device 30, a period (cycle time T) until the next transfer of the cell suspensions housed in the culture vessels 10A to 10D.

In a cell culture device 1C according to the embodiment of the disclosed technique, just as the cell culture device 1 (refer to Fig. 1) according to the first embodiment, it is possible to stir the cell suspension while suppressing damage to the cells. In addition, in the cell culture device 1C according to the embodiment, the cell suspensions housed in the culture vessels 10A and 10B are mixed in the culture vessel 10E, which is a transfer destination, and the cell suspensions housed in the culture vessels 10C and 10D are mixed in the culture vessel 10E, which is a transfer destination. Accordingly, it is possible to suppress variations in cell quality among the culture vessels.

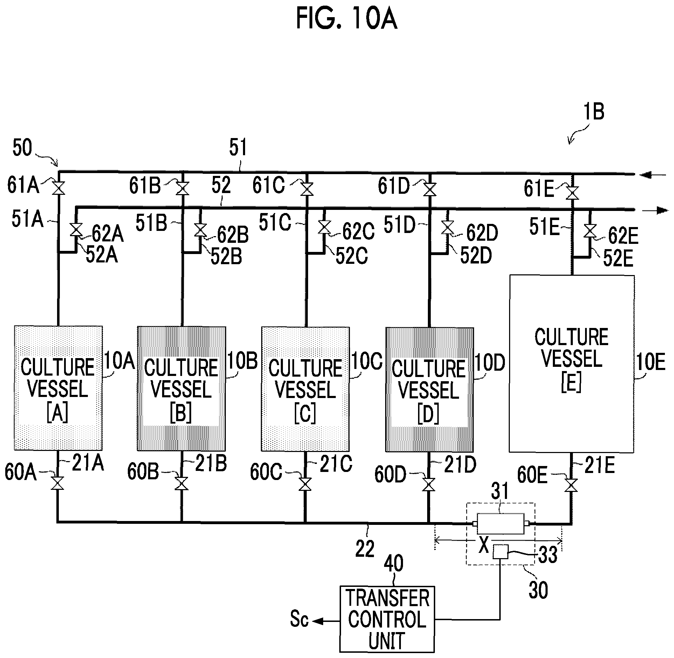

Figs. 10A to 10C are diagrams showing another example of transfer control by the transfer control unit 40 of the cell culture device 1B according to the embodiment.

In an initial state, as shown in Fig. 10A, the culture vessels 10A to 10D each house a cell suspension containing a plurality of cells (cell aggregates), and the culture vessel 10E is in an empty state. The plurality of cells (cell aggregates) are suspended in a stationary state in culture media in the culture vessels 10A to 10D. In addition, in the initial state, the valves 60A to 60E, 61A to 61E, and 62A to 62E are in a closed state.

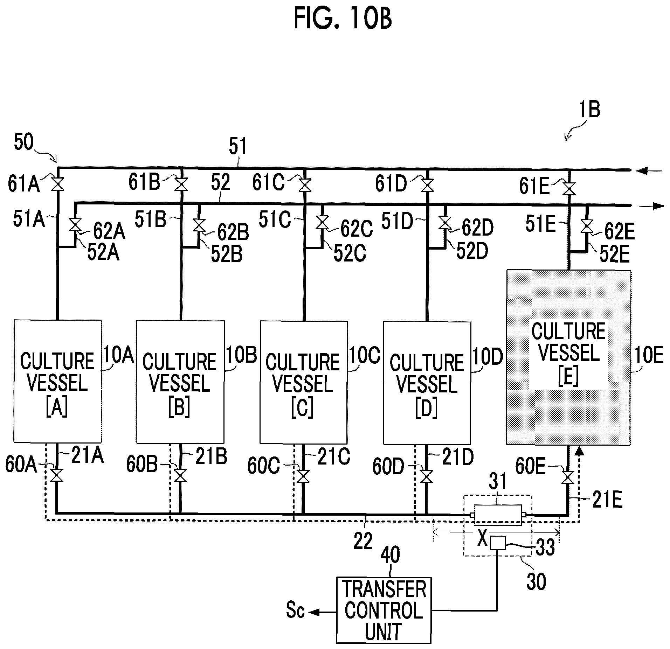

By supplying the control signal Sc to each of the valves 60A to 60F, 61A to 61D, and 62E, the transfer control unit 40 controls the valves such that the valves are in an open state. Accordingly, a gas is introduced into the culture vessels 10A to 10D via the gas introduction pipe 51, and the insides of the culture vessels 10A to 10D are pressurized. As shown in Fig. 10B, the cell suspensions housed inside the culture vessels 10A to 10D are transferred to the culture vessel 10E. While the cell suspensions pass through the section X of the common flow path 22, the cells contained in the cell suspension are monitored by the monitor device 30. That is, the imaging device 33 that composes the monitor device 30 continuously images the cells contained in the cell suspensions passing through the flow cell 31. The imaging device 33 performs imaging, for example, at an interval that allows imaging all of the cells (cell aggregates) contained in the cell suspensions, which are transfer targets. Images of the cells captured by the imaging device 33 are supplied to the transfer control unit 40. The transfer control unit 40 controls the valves 60A to 60E, 61A to 61D, and 62E such that the valves are in a closed state after the transfers of the cell suspensions are completed.

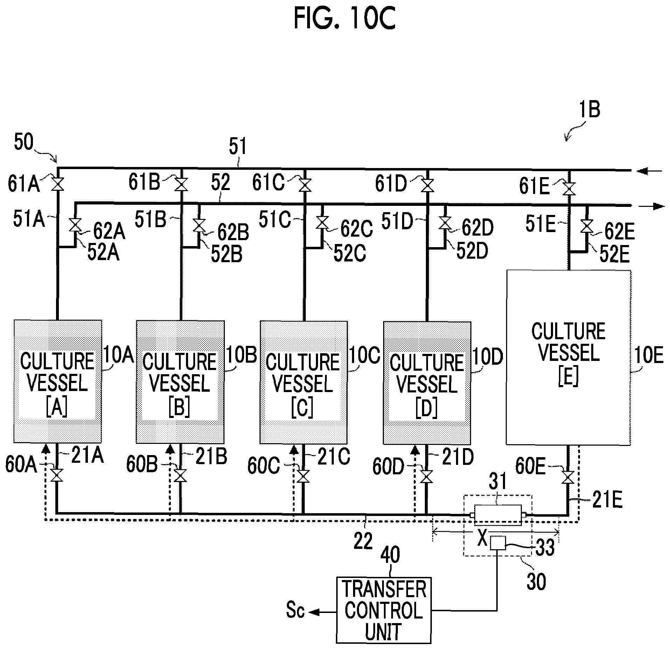

Next, the transfer control unit 40 controls the valves 60A to 60E, 62A to 62D, and 61E such that the valves are in an open state. Accordingly, a gas is introduced into the culture vessel 10E via the gas introduction pipe 51, and the inside of the culture vessel 10E is pressurized. As shown in Fig. 10C, the cell suspensions housed inside the culture vessel 10E are transferred to the culture vessels 10A to 10D. While the cell suspensions pass through the section X of the common flow path 22, the cells contained in the cell suspension are monitored by the monitor device 30. The transfer control unit 40 controls the valves 60A to 60E, 62A to 62D, and 61E such that the valves are in a closed state after the transfers of the cell suspension are completed.

As described above, in the initial state, the transfer control unit 40 performs transfer control of transferring the cell suspensions housed in the culture vessels 10A to 10D to the culture vessel 10E and then returning the cell suspensions to the culture vessels 10A to 10D. Accordingly, the transferred cell suspensions are stirred, and the nutrients and the oxygen which are contained in the culture media are distributed throughout all of the cells. Further, in the initial state, the cell suspensions housed in the culture vessels 10A to 10D are mixed in the culture vessel 10E, which is a transfer destination. Although a case where the transfer of the cell suspension between each of the culture vessels 10A to 10D and the culture vessel 10E is performed simultaneously is exemplified in the embodiment, the transfers may be performed in turn. In addition, in a case where the cell suspensions return from the culture vessel 10E to the culture vessels 10A to 10D, the monitoring of the cells by the monitor device 30 may be omitted.

The transfer control unit 40 repeatedly carries out transfer control in which one round-trip transfer of the cell suspension between each of the culture vessels 10A to 10D and the culture vessel 10E is set as one set. The transfer control unit 40 determines, based on the state of cells monitored by the monitor device 30, a period (cycle time T) until the next transfer of the cell suspensions housed in the culture vessels 10A to 10D.

In the transfer control shown in Figs. 10A to 10C, all of the cell suspensions housed in the culture vessels 10A to 10D are mixed in the culture vessel 10E, which is a transfer destination. Accordingly, an effect of suppressing variations in cell quality among the culture vessels can be further enhanced.

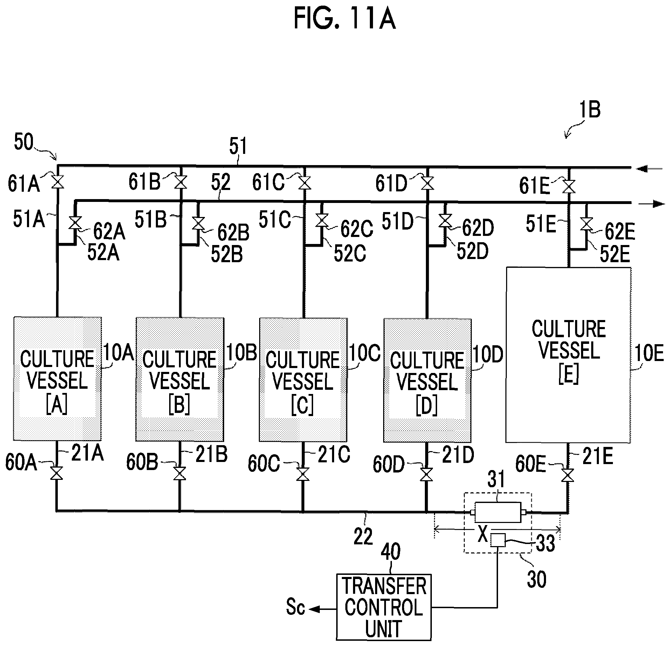

Figs. 11A to 11C are diagrams showing another example of transfer control by the transfer control unit 40 of the cell culture device I B according to the embodiment.

In an initial state, as shown in Fig. 11A, the culture vessels 10A to 10D each house a cell suspension containing a plurality of cells (cell aggregates), and the culture vessel 10E is in an empty state. The plurality of cells (cell aggregates) are suspended in a stationary state in culture media in the culture vessels 10A to 10D. In addition, in the initial state, the valves 60A to 60E, 61A to 61E, and 62A to 62E are in a closed state.

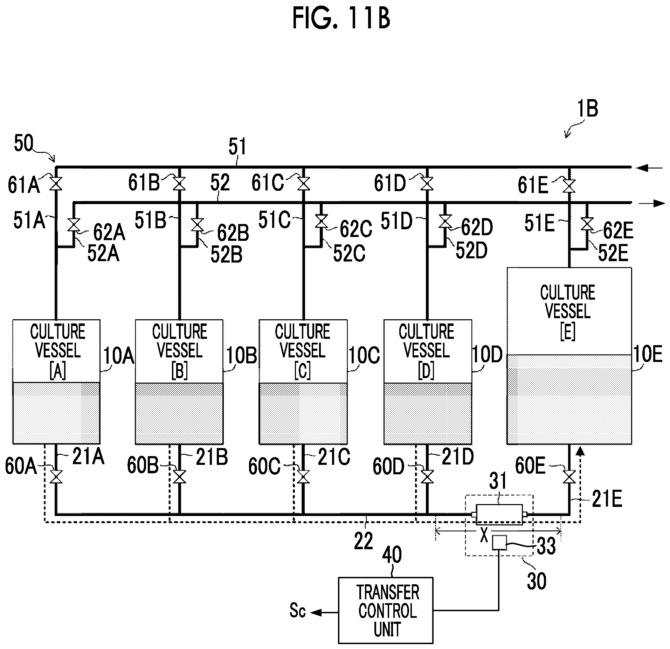

By supplying the control signal Sc to each of the valves 60A to 60E, 61A to 61D, and 62E, the transfer control unit 40 controls the valves such that the valves are in an open state. Accordingly, a gas is introduced into the culture vessels 10A to 10D via the gas introduction pipe 51, and the insides of the culture vessels 10A to 10D are pressurized. As shown in Fig. 11B, the cell suspensions housed inside the culture vessels 10A to 10D are transferred to the culture vessel 10E. In the embodiment, some (for example, half) of the cell suspension housed in each of the culture vessels 10A to 10D is transferred to the culture vessel 10E. While the cell suspensions pass through the section X of the common flow path 22, the cells contained in the cell suspension are monitored by the monitor device 30. That is, the imaging device 33 that composes the monitor device 30 continuously images the cells contained in the cell suspension passing through the flow cell 31. The imaging device 33 performs imaging, for example, at an interval that allows imaging all of the cells (cell aggregates) contained in the cell suspensions, which are transfer targets. Images of the cells captured by the imaging device 33 are supplied to the transfer control unit 40. The transfer control unit 40 controls the valves 60A to 60E, 61A to 61D, and 62E such that the valves are in a closed state after the transfers of the cell suspensions are completed.

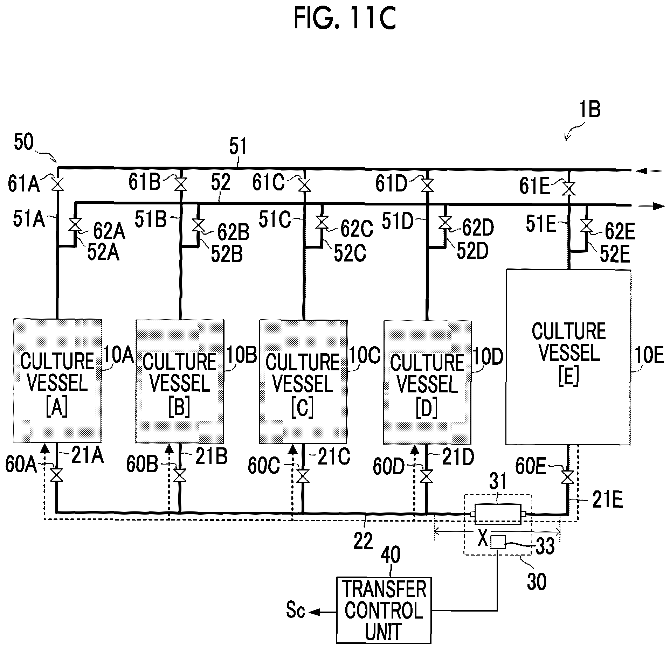

Next, the transfer control unit 40 controls the valves 60A to 60E, 62A to 62D, and 61E such that the valves are in an open state. Accordingly, a gas is introduced into the culture vessel 10E via the gas introduction pipe 51, and the inside of the culture vessel 10E is pressurized. As shown in Fig. 11C, the cell suspensions housed inside the culture vessel 10E are transferred to the culture vessels 10A to 10D. While the cell suspensions pass through the section X of the common flow path 22, the cells contained in the cell suspension are monitored by the monitor device 30. The transfer control unit 40 controls the valves 60A to 60E, 62A to 62D, and 61E such that the valves are in a closed state after the transfers of the cell suspension are completed. After transferring some of the cell suspension housed in each of the culture vessels 10A to 10D to the culture vessel 10E, the transfer control unit 40 repeatedly carries out processing of returning to the culture vessels 10A to 10D.

As described above, the transfer control unit 40 performs transfer control of reciprocating the cell suspensions housed in the culture vessels 10A to 10D in the initial state between the culture vessels 10A to 10D and the culture vessel 10E over a plurality of times. Accordingly, the transferred cell suspensions are stirred, and the nutrients and the oxygen which are contained in the culture media are distributed throughout all of the cells. Further, in the initial state, the cell suspensions housed in the culture vessels 10A to 10D are mixed in the culture vessel 10E, which is a transfer destination. Although a case where the transfers of the cell suspensions between the culture vessels 10A to 10D and the culture vessel 10E are performed simultaneously is exemplified in the embodiment, the transfers may be performed in turn. In addition, in a case where the cell suspensions return from the culture vessel 10E to the culture vessels 10A to 10D, the monitoring of the cells by the monitor device 30 may be omitted.

The transfer control unit 40 repeatedly carries out transfer control in which a predetermined times of reciprocations of the cell suspension between each of the culture vessels 10A to 10D and the culture vessel 10E is set as one set. The transfer control unit 40 determines, based on the state of cells monitored by the monitor device 30, a period (cycle time T) until the next transfer of the cell suspensions housed in the culture vessels 10A to 10D.

### [Fourth Embodiment]

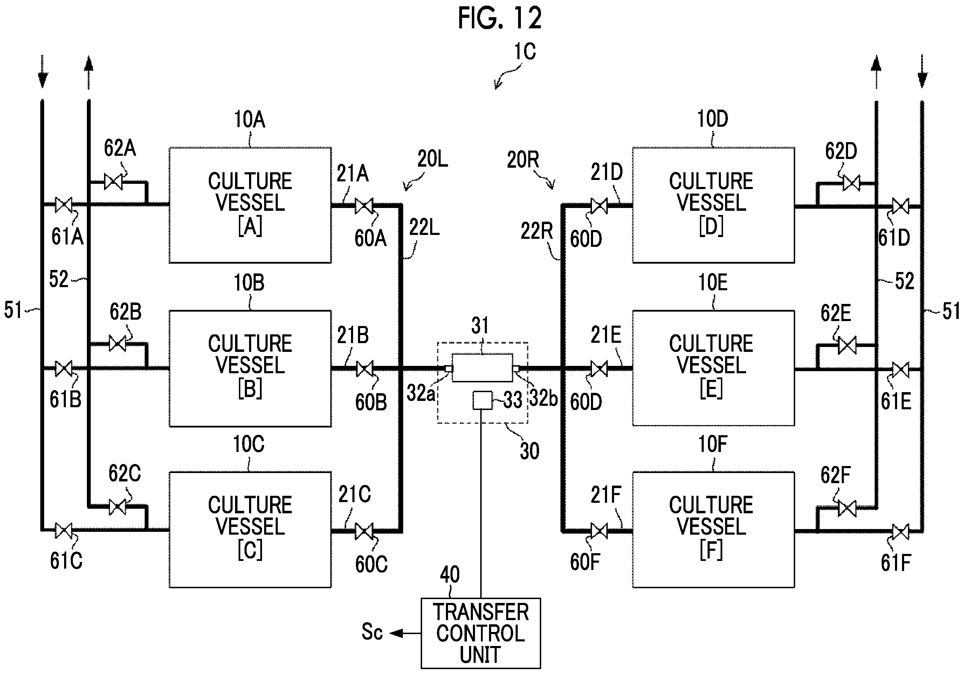

Fig. 12 is a diagram showing an example of a configuration of the cell culture device 1C according to a fourth embodiment of the disclosed technique. The cell culture device 1C comprises a plurality of culture vessels 10A, 10B, 10C, 10D, 10E, and 10F.

The culture vessels 10A to 10F are connected to each other via a first flow path 20L and a second flow path 20R. The first flow path 20L is composed of the individual flow paths 21A, 21B, and 21C, which are provided to correspond to the culture vessels 10A, 10B, and 10C respectively, and a common flow path 22L connected to the individual flow paths 21A, 21B, and 21C and the first flow port 32a of the flow cell 31. The valves 60A, 60B, and 60C are provided in the middle of the individual flow paths 21A, 21B. and 21C, respectively. The second flow path 20R is composed of the individual flow paths 21D, 21E, and 21F, which are provided to correspond to the culture vessels 10D, 10E, and 10F respectively, and a common flow path 22R connected to the individual flow paths 21D, 21E, and 21F and the second flow port 32b of the flow cell 31. The valves 60D, 60E, and 60F are provided in the middle of the individual flow paths 21D, 21E, and 21F, respectively.

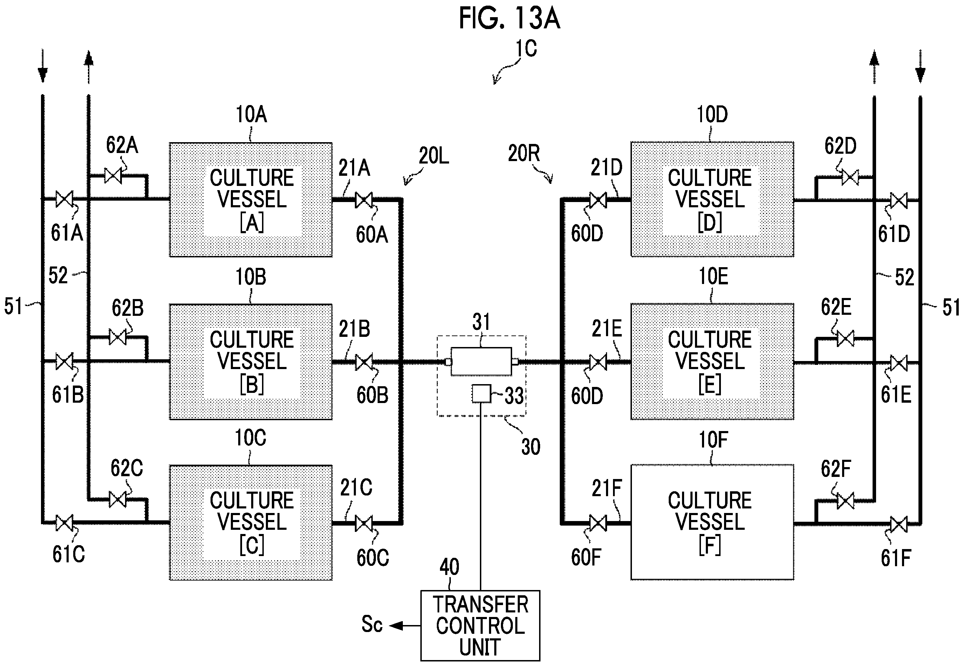

Figs. 13A to 13F are diagrams showing examples of transfer control by the transfer control unit 40 of the cell culture device 1C according to the embodiment.

In an initial state, as shown in Fig. 13A, the culture vessels 10A to 10E each house a cell suspension containing a plurality of cells (cell aggregates), and the culture vessel 10F is in an empty state. The plurality of cells (cell aggregates) are suspended in a stationary state in culture media in the culture vessels 10A to 10E. In addition, in the initial state, the valves 60A to 60F, 61A to 61F, and 62A to 62F are in a closed state.

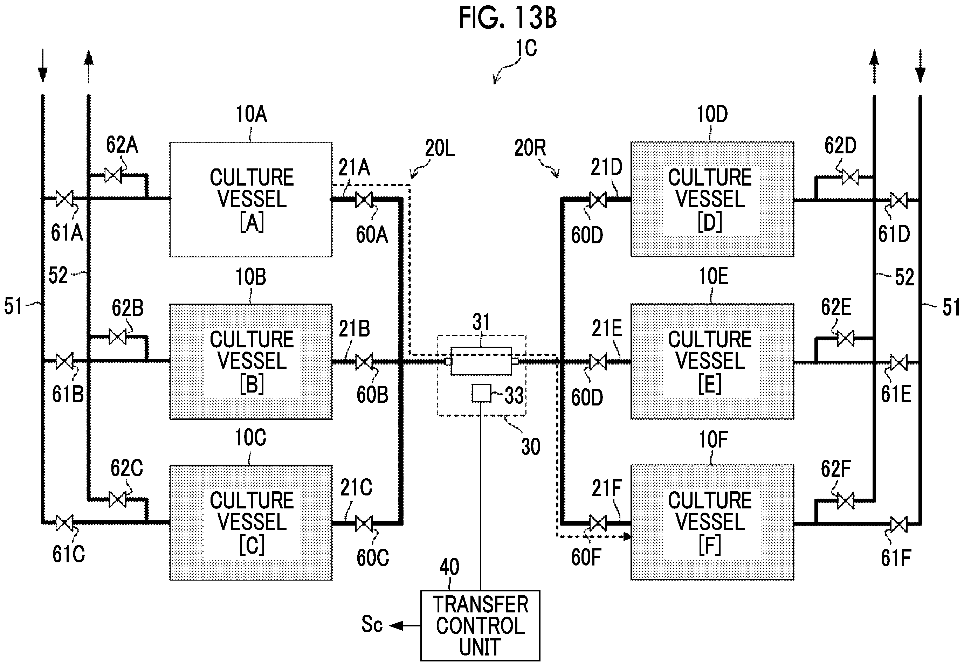

By supplying the control signal Sc to each of the valves 60A, 60F, 61A, and 62F, the transfer control unit 40 controls the valves such that the valves are in an open state. Accordingly, a gas is introduced into the culture vessel 10A via the gas introduction pipe 51, and the inside of the culture vessel 10A is pressurized. As shown in Fig. 13B, the cell suspension housed inside the culture vessel 10A is transferred to the culture vessel 10F via the first flow path 20L and the second flow path 20R. While the cell suspension passes through the section X between the first flow path 20L and the second flow path 20R, the cells contained in the cell suspension are monitored by the monitor device 30. That is, the imaging device 33 that composes the monitor device 30 continuously images the cells contained in the cell suspension passing through the flow cell 31. The imaging device 33 performs imaging, for example, at an interval that allows imaging all of the cells (cell aggregates) contained in the cell suspension, which is a transfer target. The imaging device 33 may image some cells (cell aggregates) contained in the cell suspension, which is a transfer target. Images of the cells captured by the imaging device 33 are supplied to the transfer control unit 40. The transfer control unit 40 controls the valves 60A, 60F, 61A, and 62F such that the valves are in a closed state after the transfer of the cell suspension is completed.

As described above, the transfer control unit 40 performs transfer control of transferring the cell suspension housed in the culture vessel 10A in the initial state to the culture vessel 10F. Accordingly, the transferred cell suspension is stirred, and the nutrients and the oxygen which are contained in the culture medium are distributed throughout all of the cells.

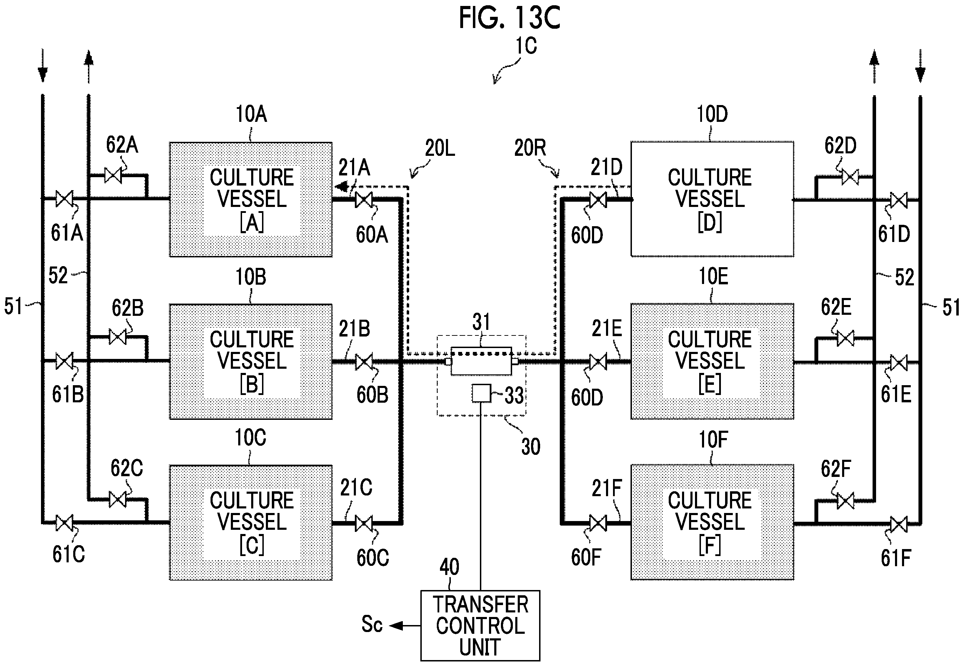

Next, by supplying the control signal Sc to each of the valves 60D, 60A, 61D, and 62A, the transfer control unit 40 controls the valves such that the valves are in an open state. Accordingly, a gas is introduced into the culture vessel 10D via the gas introduction pipe 51, and the inside of the culture vessel 10D is pressurized. As shown in Fig. 13C, the cell suspension housed inside the culture vessel 10C is transferred to the culture vessel 10A via the first flow path 20L and the second flow path 20R. While the cell suspension passes through the section X between the first flow path 20L and the second flow path 20R, the cells contained in the cell suspension are monitored by the monitor device 30. Images of the cells captured by the imaging device 33 are supplied to the transfer control unit 40. The transfer control unit 40 controls the valves 60D, 60A, 61D, and 62A such that the valves are in a closed state after the transfer of the cell suspension is completed.

As described above, the transfer control unit 40 performs transfer control of transferring the cell suspension housed in the culture vessel 10D in the initial state to the culture vessel 10A. Accordingly, the transferred cell suspension is stirred, and the nutrients and the oxygen which are contained in the culture medium are distributed throughout all of the cells.

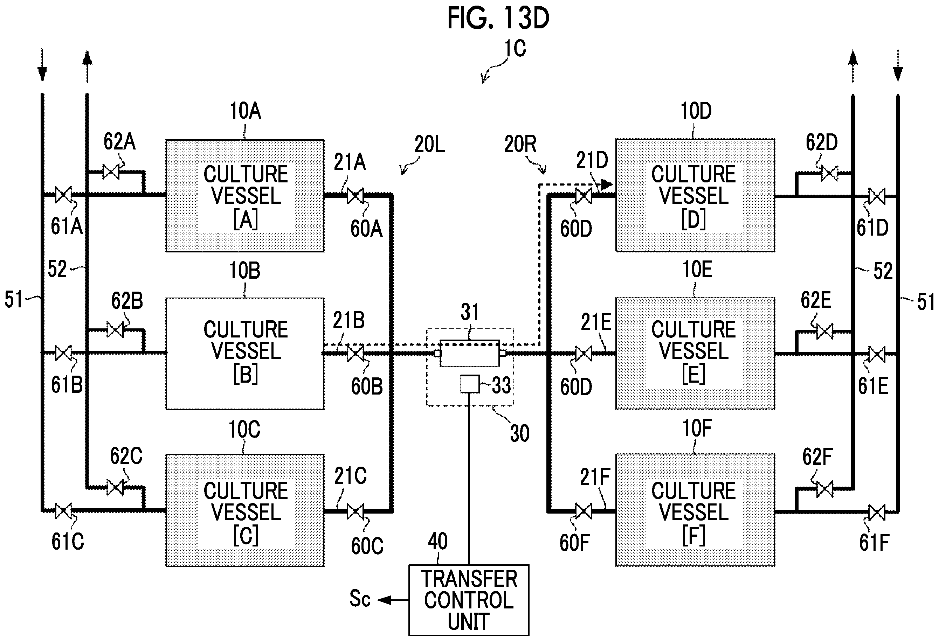

Next, by supplying the control signal Sc to each of the valves 60B, 60D, 61B, and 62D, the transfer control unit 40 controls the valves such that the valves arc in an open state. Accordingly, a gas is introduced into the culture vessel 10B via the gas introduction pipe 51, and the inside of the culture vessel 10B is pressurized. As shown in Fig. 13D, the cell suspension housed inside the culture vessel 10B is transferred to the culture vessel 10D via the first flow path 20L and the second flow path 20R. While the cell suspension passes through the section X between the first flow path 20L and the second flow path 20R, the cells contained in the cell suspension are monitored by the monitor device 30. Images of the cells captured by the imaging device 33 are supplied to the transfer control unit 40. The transfer control unit 40 controls the valves 60B, 60D, 61B, and 62D such that the valves are in a closed state after the transfer of the cell suspension is completed.

As described above, the transfer control unit 40 performs transfer control of transferring the cell suspension housed in the culture vessel 10B in the initial state to the culture vessel 10D. Accordingly, the transferred cell suspension is stirred, and the nutrients and the oxygen which are contained in the culture medium are distributed throughout all of the cells.

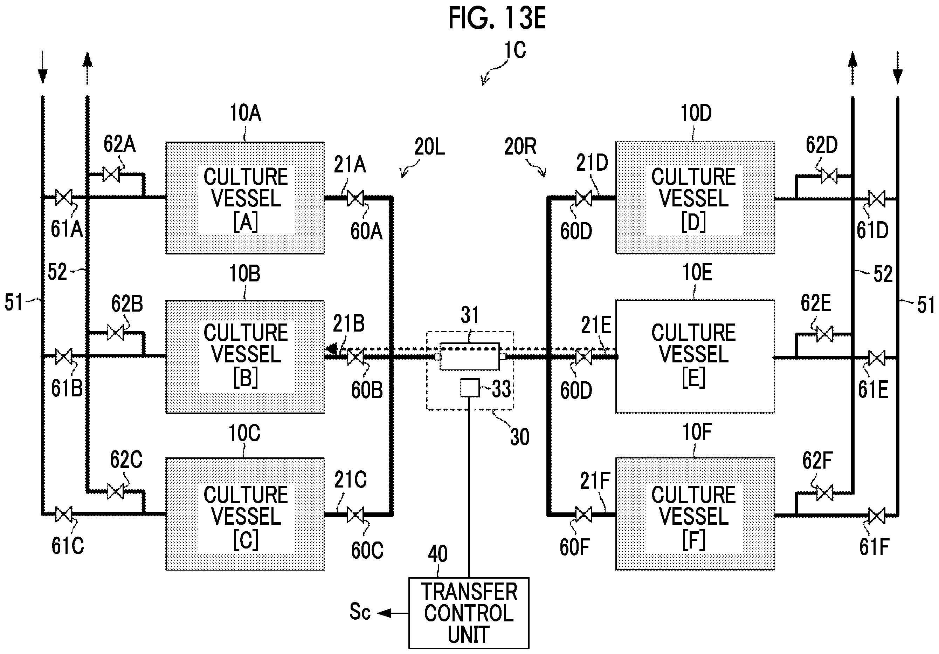

Next, by supplying the control signal Sc to each of the valves 60E, 60B, 61E, and 62B, the transfer control unit 40 controls the valves such that the valves are in an open state. Accordingly, a gas is introduced into the culture vessel 10E via the gas introduction pipe 51, and the inside of the culture vessel 10E is pressurized. As shown in Fig. 13E, the cell suspension housed inside the culture vessel 10E is transferred to the culture vessel 10B via the second flow path 20R and the first flow path 20L. While the cell suspension passes through the section X between the first flow path 20L and the second flow path 20R, the cells contained in the cell suspension are monitored by the monitor device 30. Images of the cells captured by the imaging device 33 are supplied to the transfer control unit 40. The transfer control unit 40 controls the valves 60E, 60B, 61E, and 62B such that the valves are in a closed state after the transfer of the cell suspension is completed.

As described above, the transfer control unit 40 performs transfer control of transferring the cell suspension housed in the culture vessel 10E in the initial state to the culture vessel 10B. Accordingly, the transferred cell suspension is stirred, and the nutrients and the oxygen which are contained in the culture medium are distributed throughout all of the cells.

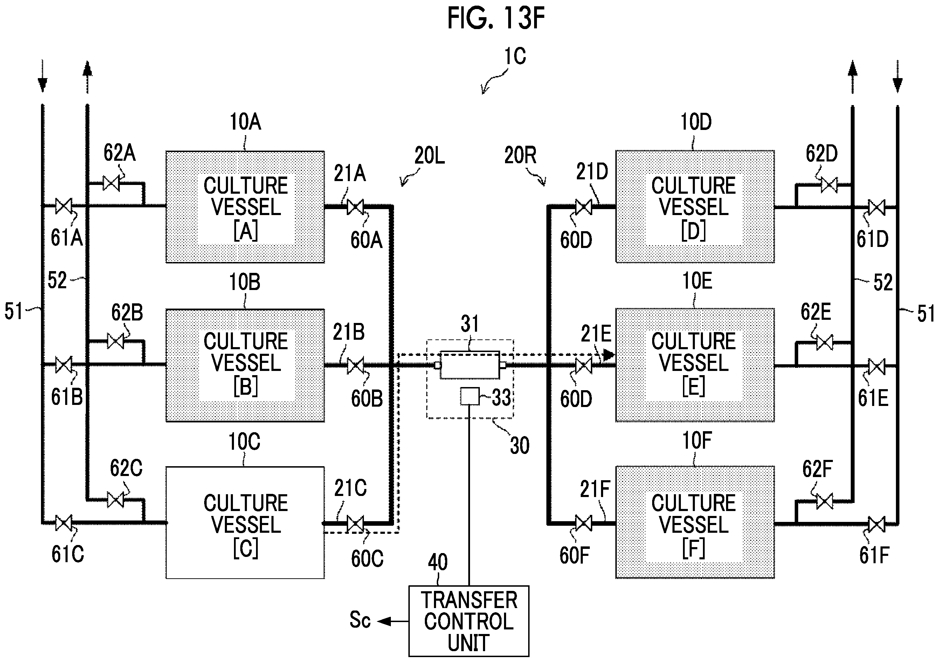

Next, by supplying the control signal Sc to each of the valves 60C, 60E, 61C, and 62E, the transfer control unit 40 controls the valves such that the valves are in an open state. Accordingly, a gas is introduced into the culture vessel 10C via the gas introduction pipe 51, and the inside of the culture vessel 10C is pressurized. As shown in Fig. 13F, the cell suspension housed inside the culture vessel 10C is transferred to the culture vessel 10E via the first flow path 20L and the second flow path 20R. While the cell suspension passes through the section X between the first flow path 20L and the second flow path 20R, the cells contained in the cell suspension are monitored by the monitor device 30. Images of the cells captured by the imaging device 33 are supplied to the transfer control unit 40. The transfer control unit 40 controls the valves 60C, 60E, 61C, and 62E such that the valves are in a closed state after the transfer of the cell suspension is completed.

As described above, the transfer control unit 40 performs transfer control of transferring the cell suspension housed in the culture vessel 10C in the initial state to the culture vessel 10E. Accordingly, the transferred cell suspension is stirred, and the nutrients and the oxygen which are contained in the culture medium are distributed throughout all of the cells.

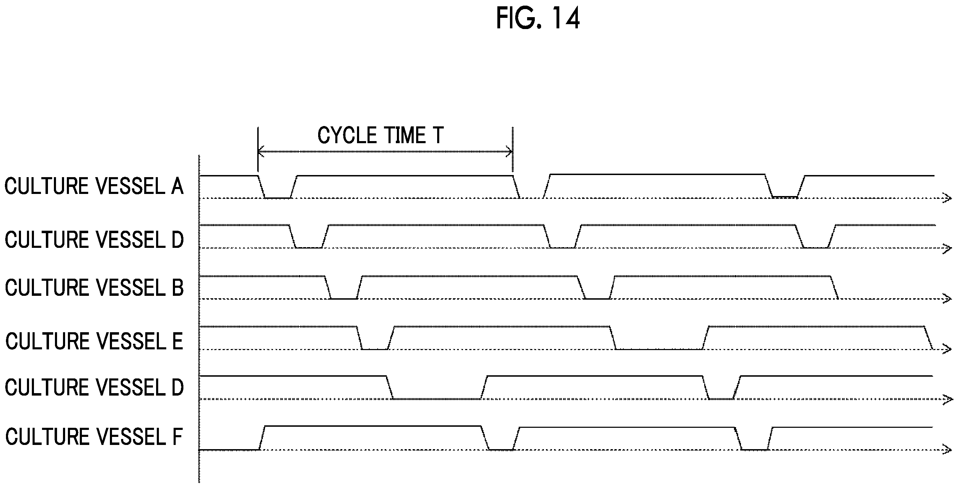

Fig. 14 is a timing chart corresponding to the series of processes of transfer control shown in Figs. 13A to 13G. In Fig. 14, "presence" and "absence" of the cell suspension are shown for each culture vessel. In Fig. 14, the "presence" of the cell suspension corresponds to a high level and the "absence" of the cell suspension corresponds to a low level. As shown in Fig. 14, the transfer control unit 40 repeatedly carries out transfer control in which one time of transfer of the cell suspension between the culture vessels 10A to 10F is set as one set. The transfer control unit 40 determines, based on a state of cells monitored by the monitor device 30, a period (cycle time T) until the next transfer of the cell suspension housed in the culture vessels 10A to 10F.

In the cell culture device 1C according to the embodiment of the disclosed technique, just as the cell culture device 1 (refer to Fig. 1) according to the first embodiment, it is possible to stir the cell suspension while suppressing damage to the cells. In addition, in the cell culture device 1C according to the embodiment, since one cycle of transfer control can be completed without reciprocating the cell suspension between any of the culture vessels 10A, 10B, or 10C connected to the first flow path 20L and any of the culture vessels 10D, 10E, or 10F connected to the second flow path 20R, it is possible to efficiently perform stirring processing of the cell suspension.

Although a case where a cell suspension is housed in one culture vessel is transferred to another culture vessel is exemplified in the embodiment, cell suspensions housed in two or more culture vessels may be transferred to another one or two or more culture vessels. Accordingly, the cell suspensions housed in the two or more culture vessels may be mixed in the culture vessel, which a transfer destination.

## Claims

1. A cell culture device comprising:
a plurality of three or more culture vessels;
a flow path that connects the plurality of culture vessels to each other; and
a transfer control unit that performs transfer control to transfer a cell suspension housed in any of the plurality of culture vessels to any other of the plurality of culture vessels via the flow path.

2. The cell culture device according to claim 1, further comprising:
a housing vessel that houses the plurality of culture vessels.

3. The cell culture device according to claim 1 or 2, further comprising:
a monitor device that is disposed at the flow path and monitors a cell contained in the cell suspension passing through the flow path.

4. The cell culture device according to claim 3,
wherein the monitor device includes an imaging device that images the cell contained in the cell suspension passing through the flow path.

5. The cell culture device according to claim 3 or 4,
wherein the transfer control unit causes the cell suspension transferred by the transfer control to pass through a part of the flow path, at which the monitor device is disposed.

6. The cell culture device according to claim 5,
wherein the transfer control unit determines, based on a state of the cell monitored by the monitor device, a period until a next transfer of the cell suspension containing the cell.

7. The cell culture device according to claim 6,
wherein as a size of a cell aggregate monitored by the monitor device is larger, the transfer control unit shortens the period until the next transfer of the cell suspension containing the cell aggregate.

8. The cell culture device according to any one of claims 3 to 7,
wherein the plurality of culture vessels comprises four or more culture vessels,
the monitor device includes a flow cell that has a first flow port and a second flow port communicating with the first flow port, and
the flow path includes a first flow path connected to the first flow port and any two or more of the plurality of culture vessels and a second flow path connected to the second flow port and any other two or more of the plurality of culture vessels.

9. The cell culture device according to any one of claims 1 to 8,
wherein in the transfer control, the transfer control unit mixes the cell suspensions housed in any two or more of the plurality of culture vessels by transferring the cell suspensions to another culture vessel of the plurality of culture vessels.

10. The cell culture device according to any one of claims 1 to 9,
wherein in the transfer control, the transfer control unit performs transfer of the cell suspension through pressure control inside the plurality of culture vessels.

11. The cell culture device according to claim 10,
wherein the pressure control is performed by introducing a gas into a culture vessel housing the cell suspension, which is a transfer target, out of the plurality of culture vessels.

12. The cell culture device according to claim 11,
wherein the gas comprises a gas whose temperature and composition are adjusted.

13. The cell culture device according to any one of claims 1 to 12, further comprising:
a heat transfer medium in contact with each of the plurality of culture vessels; and
a heat source that heats the heat transfer medium.

14. A stirring method of stirring a cell suspension housed in any of a plurality of three or more culture vessels of a cell culture device, the cell culture device including the plurality of culture vessels and a flow path that connects the plurality of culture vessels to each other, the stirring method comprising:
stirring the cell suspension, which is a transfer target, by transferring the cell suspension housed in any of the plurality of culture vessels to any other of the plurality of culture vessels via the flow path.

15. The stirring method according to claim 14, further comprising:
monitoring a cell contained in the cell suspension, which is the transfer target, while the cell suspension passes through the flow path.

16. The stirring method according to claim 15, further comprising:
determining, based on a state of the monitored cell, a period until a next transfer of the cell suspension containing the cell.

17. The stirring method according to claim 16, further comprising:
as a size of a monitored cell aggregate is larger, shortening the period until the next transfer of the cell suspension containing the cell aggregate.

18. The stirring method according to any one of claims 14 to 17, further comprising:
mixing the cell suspensions housed in any two or more of the plurality of culture vessels by transferring the cell suspensions to another culture vessel of the plurality of culture vessels.
